(19) Europäisches Patentamt
European Patent Office
Office européen des brevets

(11) **EP 3 966 348 B1**

(12) **EUROPÄISCHE PATENTSCHRIFT**

(45) Veröffentlichungstag und Bekanntmachung des Hinweises auf die Patenterteilung:
**01.01.2025 Patentblatt 2025/01**

(21) Anmeldenummer: **20726000.1**

(22) Anmeldetag: **06.05.2020**

(51) Internationale Patentklassifikation (IPC):
**C12Q 1/686** (2018.01) **B01L 7/00** (2006.01)

(52) Gemeinsame Patentklassifikation (CPC):
(C-Sets verfügbar)
**C12Q 1/686; B01L 7/52;** B01L 2400/0445 (Forts.)

(86) Internationale Anmeldenummer:
**PCT/EP2020/062531**

(87) Internationale Veröffentlichungsnummer:
**WO 2020/225286 (12.11.2020 Gazette 2020/46)**

(54) **VERFAHREN UND VORRICHTUNG ZUM EXTRAHIEREN UND/ODER VERVIELFÄLTIGEN EINER TARGET-NUKLEINSÄURE**

METHOD AND DEVICE FOR EXTRACTING AND/OR REPRODUCING A TARGET NUCLEIC ACID

PROCÉDÉ ET DISPOSITIF D'EXTRACTION ET/OU DE REPRODUCTION D'UN ACIDE NUCLEIQUE CIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.05.2019 DE 102019111841**
**24.05.2019 DE 102019114011**

(43) Veröffentlichungstag der Anmeldung:
**16.03.2022 Patentblatt 2022/11**

(73) Patentinhaber: **HP Health Solutions Germany GmbH**
**82152 Planegg/Martinsried (DE)**

(72) Erfinder:
- **SCHMIDBAUER, Simon**
  **82152 Martinsried (DE)**
- **DUCZEK, Juliane**
  **82152 Martinsried (DE)**
- **URBAN, Cordula**
  **82152 Martinsried (DE)**
- **GROTZ, Bettina**
  **82152 Martinsried (DE)**
- **MAYER, Ricarda**
  **82152 Martinsried (DE)**
- **RUSECKAS, Eimantas**
  **82152 Martinsried (DE)**
- **STEHR, Joachim**
  **82152 Martinsried (DE)**
- **BÜRSGENS, Federico**
  **82152 Martinsried (DE)**
- **ULLERICH, Lars**
  **82152 Martinsried (DE)**

(74) Vertreter: **Tautz & Schuhmacher**
**Nibelungenstraße 84**
**80639 München (DE)**

(56) Entgegenhaltungen:
**WO-A1-2018/073435 WO-A1-95/25592**

- **DAYSE. L. P. DA SILVA ET AL: "Development of capillary electrophoresis methods for quantitative determination of taurine in vehicle system and biological media", ELECTROPHORESIS, vol. 27, no. 12, 1 June 2006 (2006-06-01), Hoboken, USA, pages 2330 - 2337, XP055735069, ISSN: 0173-0835, DOI: 10.1002/ elps.200500097**
- **SIEBEN V J ET AL: "FISH and chips: chromosomal analysis on microfluidic platforms", 20070618, vol. 1, no. 3, 18 June 2007 (2007-06-18), pages 27 - 35, XP006028900**

• JOHAN VANDERHOEVEN ET AL: "Comparison of a pump-around, a diffusion-driven, and a shear-driven system for the hybridization of mouse lung and testis total RNA on microarrays", ELECTROPHORESIS, vol. 26, no. 19, 30 September 2005 (2005-09-30), pages 3773 - 3779, XP055735123, DOI: 10.1002/ elps.200500097
• ERICKSON D ET AL: "ELECTROKINETICALLY CONTROLLED DNA HYBRIDIZATION MICROFLUIDIC CHIP ENABLING RAPID TARGET ANALYSIS", ANALYTICAL CHEMISTRY, AMERICAN CHEMICAL SOCIETY, vol. 76, no. 24, 15 December 2004 (2004-12-15), pages 7269 - 7277, XP001226129, ISSN: 0003-2700, DOI: 10.1021/AC049396D

(52) Gemeinsame Patentklassifikation (CPC): (Forts.)

C-Sets
**C12Q 1/686, C12Q 2523/307, C12Q 2527/101, C12Q 2563/137, C12Q 2565/518; C12Q 1/686, C12Q 2565/518, C12Q 2565/629**

**Beschreibung**

Die vorliegende Erfindung betrifft Verfahren und eine Vorrichtung zum Extrahieren und/oder Vervielfältigen bzw. Amplifizieren einer Target-Nukleinsäure. Die Erfindung liegt somit auf dem Gebiet der Molekulardiagnostik und insbesondere auf dem Gebiet der Extraktion und/oder Aufreinigung und/oder Amplifikation von Nukleinsäuren, letzteres beispielsweise mittels einer Polymerase-Kettenreaktion (PCR).

Stand der Technik

Im Stand der Technik sind Verfahren bekannt, bei welchen mittels lokalem Heizen eine Polymerase-Kettenreaktion durchgeführt wird, um eine Nukleinsäure in einer Reaktionslösung zu vervielfältigen bzw. zu amplifizieren. Ein solches Verfahren ist insbesondere aus der Druckschrift DE 10 2016 120 124 A1 bekannt. Dabei sind als Heizdrähte ausgebildete Heizelemente mit Oligonukleotiden funktionailisiert, mit welchen die zu amplifizierende Target-Nukleinsäure an die jeweiligen Heizelemente gebunden werden kann. Beispielsweise können die Oligonukleotide als Primeroligonukleotide für die Polymerase-Kettenreaktion ausgebildet sein, wenngleich dies nicht zwingend erforderlich ist.

Damit eine frei in der Reaktionslösung befindliche Target-Nukleinsäure an eines der an ein Heizelement funktionalisiertes Oligonukleotid binden bzw. hybridisieren kann, ist es neben einer geeigneten chemischen Umgebung und einer geeigneten Temperatur erforderlich, dass sich die Target-Nukleinsäure in einer ausreichend geringen räumlichen Nähe zu dem Oligonukleotid befindet.

Eine ausreichend geringe räumliche Nähe ist auch erforderlich, um beispielsweise eine Target-Nukleinsäure, die frei in einer Probenflüssigkeit vorliegt, zu extrahieren, indem diese beispielsweise an eine Extraktionsnukleinsäure hybridisiert, welches an ein Heizelement oder ein anderes Substrat funktionalisiert ist.

Der Prozess des räumlichen Zusammenbringens von Target-Nukleinsäure und Funktions- bzw. Extraktionsnukleinsäure bzw. Primer auf den Heizelementen folgt herkömmlicherweise im Wesentlichen einer Brownschen Molekularbewegung bzw. durch ungerichtete Diffusion. Bei den im Stand der Technik bekannten Verfahren erlaubt die Brownsche Molekularbewegung bzw. die Diffusion typischerweise kein schnelles Zusammenbringen von Target-Nukleinsäure und Funktionsnukleinsäure bzw. Primer auf den Heizelementen, die in vielen bevorzugten Ausführungsformen nur an diskreten Orten lokalisiert vorliegen und anders als z.B. freie Primer nicht homogen über das Volumen der Probenflüssigkeit verteilt sind. Dies führt bei herkömmlichen Verfahren dazu, dass unnötig lang gewartet werden muss, bis eine ausreichende Menge von Target-Nukleinsäuren die Funktionsnukleinsäure bzw. Primer auf den Heizelementen gefunden hat, insbesondere wenn die Ausgangskonzentration der zu vervielfältigenden Nukleinsäure sehr gering ist. Dies verlängert dabei unter Umständen unnötig den Gesamtprozess des Nachweises und der Charakterisierung von Nukleinsäuren. Es kann auch dazu führen, dass, wenn nicht ausreichend lang gewartet wird, bis eine ausreichende Menge von Target-Nukleinsäuren die Funktionsnukleinsäure bzw. Primer auf den Heizelementen gefunden hat, zu Beginn der Amplifikationsreaktion gar keine Nukleinsäure (oder zu wenige) auf die Funktionsnukleinsäure bzw. Primer auf den Heizelementen gebunden hat und die Target-Nukleinsäure somit unter Umständen nicht (oder nicht zuverlässig) in der Amplifikationsreaktion nachgewiesen oder charakterisiert werden kann, obwohl sie in der Ausgangsflüssigkeit, die mit den Heizelementen bzw. den Funktionsnukleinsäuren bzw. Primern auf den Heizelementen in Kontakt gebracht wurde, vorhanden war.

Die Nachweis- oder Charakterisierungsreaktion kann somit falsche Ergebnisse liefern, nur zu einem geringen Prozentsatz richtige Ergebnisse liefern oder Ergebnisse unnötig spät liefern.

Je geringer die Konzentration der Target-Nukleinsäure in der Probenflüssigkeit und/oder in der Reaktionslösung ist, desto größer ist auch der mittlere Abstand zwischen der Target-Nukleinsäure und einer Funktionsnukleinsäure bzw. einem Heizelement. Entsprechend kann bei geringen Konzentrationen von Target-Nukleinsäuren in der Probenflüssigkeit und/oder der Reaktionslösung eine erhebliche Zeitdauer erforderlich sein, bis die Target-Nukleinsäure in ausreichendem Maße an die Oligonukleotide bzw. Funktionsnukleinsäuren hybridisiert sind, um eine effiziente Extraktion und/oder Vervielfältigung der Target-Nukleinsäure zu erreichen.

WO2018073435A1 beschreibt ein PCR-Verfahren, bei dem das Reaktionsvolumen durch den Einsatz von weniger als 20 Joule pro Milliliter elektrischer Energie erhitzt wird.

WO95/25592 beschreibt ein Verfahren zur Verarbeitung von Nukleinsäuren in einem Reaktionsgemisch auf einer Oberfläche und deren unmittelbarer Umgebung mit einer regelbaren Temperatur, bei der die Hauptkammer des Reaktionsgemisches im Wesentlichen isotherm bleibt.

Es ist daher die Aufgabe der Erfindung, ein Verfahren und eine Vorrichtung bereitzustellen, die die Extraktions- und/oder Amplifikationseffizienz und/oder die Sensitivität zu erhöhen und/oder eine dafür erforderliche Zeitdauer verringern.

Offenbarung der Erfindung

In einem ersten Aspekt betrifft die Erfindung ein Verfahren zum Extrahieren einer Target-Nukleinsäure aus einer Probenflüssigkeit. Das Verfahren umfasst ein Bereitstellen einer Heizeinrichtung mit einem Heizelement in Kontakt mit der Probenflüssigkeit, wobei das Heizelement mit zumindest einer Funktionsnukleinsäure konjugiert ist und wobei die zumindest eine Funktionsnukleinsäure dazu ausgelegt ist, mit der Target-Nukleinsäure zu hybridisieren und die Target-Nukleinsäure an das Heizelement zu binden. Ferner umfasst das Verfahren ein Erzeugen einer Relativbewegung zwischen dem Heizelement und der Probenflüssigkeit sowie ein Extrahieren der Target-Nukleinsäure aus der Probenflüssigkeit durch ein Separieren des Heizelements von der Probenflüssigkeit.

In einem weiteren Aspekt betrifft die Erfindung ein Verfahren zum Vervielfältigen einer Target-Nukleinsäure in einer Reaktionslösung, umfassend ein Bereitstellen einer Heizeinrichtung mit einem Heizelement in Kontakt mit der Reaktionslösung, wobei das Heizelement mit zumindest einer Funktionsnukleinsäure konjugiert ist und wobei die zumindest eine Funktionsnukleinsäure dazu ausgelegt ist, mit der Target-Nukleinsäure zu hybridisieren. Ferner umfasst das Verfahren ein Erzeugen einer Relativbewegung zwischen dem Heizelement und der Reaktionslösung, sowie ein Vervielfältigen der Target-Nukleinsäure mittels einer Polymerase-Kettenreaktion in der Reaktionslösung unter Verwendung der Heizeinrichtung.

In einem weiteren Aspekt betrifft die Erfindung ein Verfahren zum Vervielfältigen einer Target-Nukleinsäure, umfassend ein Extrahieren der Target-Nukleinsäure aus einer Probenflüssigkeit durch Bereitstellen einer Heizeinrichtung mit einem Heizelement in Kontakt mit der Probenflüssigkeit, wobei das Heizelement mit zumindest einer Funktionsnukleinsäure konjugiert ist und wobei die zumindest eine Funktionsnukleinsäure dazu ausgelegt ist, mit der Target-Nukleinsäure zu hybridisieren und die Target-Nukleinsäure an das Heizelement zu binden. Ferner umfasst das Verfahren ein Vervielfältigen der aus der Probenflüssigkeit extrahierten Target-Nukleinsäure mittels einer Polymerase-Kettenreaktion in einer Reaktionslösung unter Verwendung der Heizeinrichtung. Dabei umfasst das Extrahieren der Target-Nukleinsäure ferner ein Erzeugen einer Relativbewegung zwischen dem Heizelement und der Probenflüssigkeit. Alternativ oder zusätzlich umfasst das Vervielfältigen der Target-Nukleinsäure ferner ein Erzeugen einer Relativbewegung zwischen dem Heizelement und der Reaktionslösung.

In einem weiteren Aspekt betrifft die Erfindung eine Vorrichtung zum Extrahieren und/oder Vervielfältigen einer Target-Nukleinsäure. Die Vorrichtung umfasst eine Heizeinrichtung mit einem Heizelement, wobei das Heizelement mit einer Probenflüssigkeit und/oder mit einer Reaktionslösung in Kontakt bringbar ist und mit zumindest einer Funktionsnukleinsäure konjugiert ist, wobei die zumindest eine Funktionsnukleinsäure dazu ausgelegt ist, mit der Target-Nukleinsäure zu hybridisieren und die Target-Nukleinsäure an das Heizelement zu binden. Zudem umfasst die Vorrichtung eine Bewegungseinrichtung, welche dazu ausgelegt ist, eine Relativbewegung zwischen dem zumindest einen Heizelement und der Reaktionslösung und/oder der Probenflüssigkeit zu erzeugen, wenn das Heizelement in Kontakt mit der Reaktionslösung bzw. der Probenflüssigkeit ist.

Die Erfindung bietet den Vorteil, dass das Zusammenfinden von Target-Nukleinsäure und Funktionsnukleinsäure bzw. Heizelement begünstigt und beschleunigt werden kann. Durch die Relativbewegung zwischen Heizelement und Probenflüssigkeit bzw. Reaktionslösung kann effektiv das Volumen, mit dem das Heizelement in Kontakt ist, erhöht werden, sodass auch die Wahrscheinlichkeit, dass eine Target-Nukleinsäure in ausreichend geringem Abstand zu einer Funktionsnukleinsäure gelangt, um damit zu hybridisieren, erhöht wird. Auf diese Weise kann mit einfachen und kostengünstigen Mitteln das Extrahieren und/oder Amplifizieren der Target-Nukleinsäure beschleunigt werden und/oder die Sensitivität erhöht werden.

Die Erfindung bietet ferner den Vorteil, dass sich dadurch die Zeitdauer für die Durchführung von Polymerase-Kettenreaktionen, die mit lokalem Heizen durchgeführt werden und beispielsweise weiter oben und in der DE 10 2016 120 124 A1 beschrieben sind, noch weiter reduziert werden kann und/oder die Effizienz und/oder Sensitivität erhöht werden kann. Insbesondere kann die Erfindung den Vorteil bieten, dass bei einer Polymerase-Kettenreaktion auf Basis von lokalem Heizen bei Verwendung des erfindungsgemäßen Verfahrens geringere Ausgangskonzentrationen der Target-Nukleinsäure ausreichend sein können, da durch die Relativbewegung die Wahrscheinlichkeit für ein Zusammenfinden der Target-Nukleinsäuren mit einer Funktionsnukleinsäure erhöht wird. Somit kann mit der Erfindung die Sensitivität für den Nachweis von Target-Nukleinsäuren und deren Vervielfältigung noch weiter verbessert werden.

Ferner bietet die Erfindung den Vorteil, dass die Hybridisierung der Target-Nukleinsäure an eine Funktionsnukleinsäure nicht durch die Diffusion und/oder Brownsche-Bewegung der Target-Nukleinsäure in der Probenflüssigkeit und/oder Reaktionslösung limitiert ist und somit die Sensitivität und Schnelligkeit der Extraktion und/oder Amplifikation gesteigert werden kann.

Die Probenflüssigkeit ist vorzugsweise Ausgangsflüssigkeit, die die Target-Nukleinsäure enthält (z.B. Probenmaterial, das die Target-Nukleinsäure enthält, oder Probenmaterial, in der die Target-Nukleinsäure (zuvor) freigesetzt wurde (z.B. aus Pathogenen und/oder Zellen) oder eine Flüssigkeit, in der die Nukleinsäure bereits im aufgereinigtem Zustand vorliegt). Die Probenflüssigkeit kann vorzugsweise auch Reagenzien aufweisen oder mit solchen versehen werden, die das Hybridisieren der Target-Nukleinsäure an die Funktionsnukleinsäure bzw. Extraktionsnukleinsäure bzw. das Primeroligonukleotid auf dem Heizelement begünstigt, wie etwa entsprechende Salze.

Die Probenflüssigkeit ist vorzugsweise eine Flüssigkeit, welche gegebenenfalls die Target-Nukleinsäure aufweist und auch noch andere Bestandteile umfasst. Insbesondere kann die Probenflüssigkeit noch weitere Bestandteile aufweisen, welche nicht mit extrahiert werden sollen. Diese anderen Bestandteile können andere Nukleinsäuren umfassen, also Nukleinsäuren, welche eine andere Nukleotidsequenz aufweisen. Auch können die anderen Bestandteile Verunreinigungen aufweisen, welche von nicht-nukleotidischer Art sind. Insbesondere kann es sich bei der Probenflüssigkeit um eine Flüssigkeit handeln, welche beispielsweise menschlichen und/oder tierischen und/oder pflanzlichen und/oder anderweitigen organischen Ursprungs ist. Beispielsweise kann die Probenflüssigkeit Blut und/oder Sekret und/oder Körperausscheidungen und/oder Absonderungen von Schleimhäuten und/oder Speichel und/oder Zellflüssigkeit enthalten oder daraus bestehen. Die Probenflüssigkeit kann vorzugsweise vor dem Extrahieren einer oder mehreren Behandlungen unterzogen worden sein, um etwa in der Probenflüssigkeit enthaltene Nukleinsäuren zumindest teilweise freizusetzen. Beispielsweise kann die Probenflüssigkeit einer Behandlung unterzogen worden sein, um etwa darin befindliche Zellen zu lysieren bzw. aufzuschließen, um die ggf. darin befindlichen Nukleinsäuren zumindest teilweise aus den Zellen herauszulösen, sodass die Nukleinsäuren vorzugsweise frei in der Probenflüssigkeit vorliegen und zumindest teilweise nicht oder nicht mehr in Zellkernen und/oder Zellen eingeschlossen sind. Das Lysieren erfolgt vorzugsweise derart, dass die freigesetzten Nukleinsäuren zumindest nicht vollständig zerstört werden und besonders bevorzugt vollständig erhalten bzw. unversehrt bleiben.

Insbesondere kann die Probenflüssigkeit vorzugsweise derart vorliegen, dass ein Durchführen einer Amplifikationsreaktion zur Vervielfältigung der extrahierten Nukleinsäure in der Probenflüssigkeit nicht möglich ist. So kann die Probenflüssigkeit beispielsweise physikalische und/oder chemische und/oder biologische Eigenschaften aufweisen, welche einer Durchführung einer Amplifikationsreaktion, wie beispielsweise einer PCR, in der Probenflüssigkeit entgegenstehen. Beispielsweise kann die Probenflüssigkeit eine Viskosität und/oder einen pH-Wert und/oder eine Salzkonzentration und/oder Polarität und/oder Enzyme und/oder Proteasen aufweisen, welche die Durchführung einer Amplifikationsreaktion nicht ermöglichen, beispielsweise weil die Aktivität der hierzu notwendigen Polymerase-Enzyme inhibiert wird oder Enzyme wie Proteasen vorliegen, die die Polymerase-Enzyme abbauen können.

Vorzugsweise kann zumindest ein Parameter der Probenflüssigkeit angepasst werden, um ein Hybridisieren der Zielnukleinsäure an die Funktionsnukleinsäure angesichts des vorliegenden Maßes an Komplementarität zu ermöglichen. Beispielsweise kann eine Konzentration von $MgCl_2$ in der Probenflüssigkeit erhöht werden, um auch ein Hybridisieren bei geringer Komplementarität zu ermöglichen, wohingegen vorzugsweise die Konzentration von $MgCl_2$ in der Probenflüssigkeit verringert werden kann, um ein Hybridisieren erst ab einem bestimmten höheren Maß an Komplementarität zu ermöglichen.

Vorzugsweise erfolgt ein In-Kontakt-Bringen des Heizelements mit der Probenflüssigkeit derart, dass die aus der Probenflüssigkeit zu extrahierende Nukleinsäure zumindest teilweise an die Funktionsnukleinsäure bindet. Dies kann umfassen, dass zumindest an der Kontaktfläche zwischen dem Heizelement und der Probenflüssigkeit, also an der Oberfläche des Heizelements und/oder in der unmittelbaren Umgebung, zumindest teilweise Hybridisierungsbedingen vorherrschen, welche ein Hybridisieren der zu extrahierenden Nukleinsäure mit der Extraktionsnukleinsäure begünstigen und/oder ermöglichen. Beispielsweise können die Hybridisierungsbedingungen eine Temperatur und/oder einen pH-Wert und/oder eine Salzkonzentration in einem bestimmten Bereich umfassen. Gemäß einer bevorzugten Ausführungsform kann das Heizelement dabei derart beheizt werden, dass in der unmittelbaren Umgebung zumindest zeitweise die erforderliche Temperatur bereitgestellt wird, um die Hybridisierung der zu extrahierenden Nukleinsäure mit der Funktionsnukleinsäure zu ermöglichen. Gemäß einer weiteren bevorzugten Ausführungsform, kann das Heizelement und die Probenflüssigkeit vorzugsweise auch möglichst homogen beheizt werden, d.h. derart beheizt werden, dass die Heizelemente die gleiche oder nahezu die gleiche Temperatur aufweisen wie die Reaktionslösung, um eine effektive Hybridisierung der zu extrahierenden Nukleinsäure an die Funktionsnukleinsäure zu ermöglichen. Dies kann vorzugsweise durch das Heizelement und/oder durch eine optionale weitere, vorzugsweise außerhalb der Probenflüssigkeit angeordneten Heizvorrichtung, wie etwa einen Temperierblock, realisiert werden. Hierzu kann beispielsweise die gesamte Vorrichtung, die die Heizelemente sowie die Probeflüssigkeit enthält, auf eine Basistemperatur von beispielsweise zwischen 35°C und 80° und besonders vorzugsweise zwischen 40°C und 70°C aufgeheizt werden. Eine derartige Basistemperatur kann vorzugsweise zumindest zeitweise während der Extraktion eingestellt werden. Während einer optionalen Amplifikation nach der Extraktion kann optional vom Einstellen derselben Basistemperatur abgesehen werden und/oder eine andere Basistemperatur bzw. globale Temperatur der Reaktionslösung eingestellt werden.

Die Reaktionslösung weist vorzugsweise Reagenzien auf, die zur Vervielfältigung mittels Polymerase Kettenreaktion nötig sind und kann mit den Heizelementen bzw. den Funktionsnukleinsäuren bzw. Primern auf den Heizelementen in Kontakt gebracht werden, um ein Binden der Target-Nukleinsäure auf die Funktionsnukleinsäure auf den Heizelementen zu ermöglichen.

Die Reaktionslösung ist dabei vorzugsweise eine Flüssigkeit, in welcher die Target-Nukleinsäure als solche einzelsträngig und/oder doppelsträngig vorliegend überdauern kann und oder stabilisiert wird. Jedoch unterscheidet sich die Reaktionslösung von der Probenflüssigkeit. Mit anderen Worten ist die Reaktionslösung vorzugsweise derart ausgestaltet, dass die Target-Nukleinsäure durch die Wechselwirkung mit der Reaktionslösung keinen Schaden nimmt. Beispielsweise kann die Reaktionslösung als eine wässrige Lösung und/oder als eine Pufferlösung vorliegen. Sofern nach dem Extrahieren der Target-Nukleinsäure eine Amplifikation der Target-Nukleinsäure beabsichtigt ist, kann die Reaktionslösung vorzugsweise derart ausgestaltet sein, dass die Reaktionslösung die Durchführung einer solchen Amplifikationslösung ermöglicht. Beispielsweise kann die Reaktionslösung als eine Pufferlösung ausgebildet sein, in welcher die Durchführung einer PCR möglich ist, um die Target-Nukleinsäure zumindest teilweise zu vervielfältigen. Die Reaktionslösung wird vorzugsweise in einem Reaktionsgefäß bereitgestellt. Die bereitgestellte Reaktionslösung weist vorzugsweise ein Volumen von zumindest 1 µl und nicht mehr als 10 ml, mehr bevorzugt von zumindest 5 µl und nicht mehr als 1 ml, am meisten bevorzugt von zumindest 10 µl und nicht mehr als 100 µl auf.

Die Bezeichnungen "Nukleinsäure" und "Oligonukleotid" umfassen im Zusammenhang mit der vorliegenden Erfindung nicht nur (Desoxy)-Ribonukleinsäuren bzw. (Desoxy)-Oligo-Ribonukleotide, auch wenn vorgenannte bevorzugt sind, sondern auch Nukleinsäuren und Oligonukleotide, die ein oder mehrere Nukleotid-Analoga mit Modifikationen an ihrem Backbone (zum Beispiel Methylphosphonate, Phosphothioate oder Peptide Nucleic Acids (PNA), insbesondere an einem Zucker des Backbone (zum Beispiel 2'-O-Alkylderivate, 3'- und/oder 5'-Aminoribosen, Locked Nucleic Acids [LNA], Hexitol Nucleic Acids, Morpholinos, Glycol Nucleic Acid (GNA), Threose Nucleic Acid (TNA) oder Tricyclo-DNA, vergleiche hierzu den Aufsatz von D. Renneberg und C.J. Leumann, "Watson-Crick base-pairing properties of Tricyclo-DNA", J. Am. Chem. Soc., 2002, Bd. 124, Seiten 5993-6002, dessen diesbezüglicher Inhalt durch Verweis Teil der vorliegenden Offenbarung ist) enthalten oder die Basen-Analoga enthalten, zum Beispiel 7-Deazapurine oder Universalbasen wie Nitroindol oder modifizierte natürliche Basen wie N4-Ethyl-Cytosin. In einer Ausführung der Erfindung sind die Nukleinsäuren oder Oligonukleotide Konjugate oder Chimären mit nichtnukleosidischen Analoga, zum Beispiel PNA. Die Nukleinsäuren oder Oligonukleotide enthalten in einer Ausführung der Erfindung an einer oder mehreren Positionen nicht-nukleosidische und/oder nicht-nukleotidische Einheiten wie Spacer, zum Beispiel Hexaethylenglycol oder Cn-Spacer mit n zwischen 3 und 6. Soweit die Nukleinsäuren oder Oligonukleotide Modifikationen enthalten, sind diese so gewählt, dass auch mit der Modifikation eine Hybridisierung mit natürlichen DNA/RNA-Analyten möglich ist. Bevorzugte Modifikationen beeinflussen das Schmelzverhalten, vorzugsweise die Schmelztemperatur, insbesondere um Hybride mit unterschiedlichen Graden der Komplementarität ihrer Basen unterscheiden zu können (Mismatch-Diskriminierung). Bevorzugte Modifikationen umfassen LNA, 8-Aza-7-Deaza- Purine, 5-Propinyl-Uracil und - Cytosin und/oder abasische Unterbrechungen oder Modifikationen in der Nukleinsäure oder in dem Oligonukleotid. Weitere Modifikationen im Sinne der Erfindung sind zum Beispiel Modifikationen mit Biotin und/oder Thiol und/oder Schwefel und/oder Fluoreszenzdonor- und Fluoreszenzakzeptormolekülen.

Die Begriffe Target-Nukleinsäure und Nukleinsäure werden im Rahmen dieses Dokuments als Äquivalente verwendet, sofern nicht anders angegeben. Dass die Nukleinsäure aus der Probenflüssigkeit extrahiert wird, bedeutet dabei, dass die Nukleinsäure zumindest teilweise von der Probenflüssigkeit isoliert wird und vorzugsweise von der Probenflüssigkeit getrennt werden kann. Die aus der Probenflüssigkeit zu extrahierende Nukleinsäure kann auch als Zielnukleinsäure oder Target-Nukleinsäure bezeichnet werden. Insbesondere kann somit das Extrahieren einer Nukleinsäure ein Separieren der Nukleinsäure von der Probenflüssigkeit umfassen oder einem solchen Separieren dienen. Dabei kann das Extrahieren der Nukleinsäure derart ausgestaltet sein, dass lediglich die zu extrahierende Nukleinsäure von der Probenflüssigkeit extrahiert wird oder auch noch andere Bestandteile der Probenflüssigkeit mit extrahiert werden, wie beispielsweise andere Nukleinsäuren. Besonders bevorzugt wird jedoch nur die zu extrahierende Nukleinsäure aus der Probenflüssigkeit extrahiert, sodass insbesondere andere Nukleinsäuren und andere Bestandteile der Probenflüssigkeit nicht mit extrahiert werden, sondern in der Probenflüssigkeit verbleiben. Vorzugsweise ist nach dem Extrahieren der Nukleinsäure die Konzentration und/oder die Anzahl von Exemplaren der extrahierten Nukleinsäure in der Probenflüssigkeit geringer, als vor dem Extrahieren, sofern überhaupt vor dem Extrahieren die zu extrahierende Nukleinsäure in der Probenflüssigkeit vorhanden war.

Die Funktionsnukleinsäure ist vorzugsweise zumindest teilweise komplementär zur Target-Nukleinsäure. Dass die Funktionsnukleinsäure zumindest teilweise komplementär zur Zielnukleinsäure ist, bedeutet dabei, dass die Funktionsnukleinsäure und die Zielnukleinsäure über zumindest ein Basenpaar miteinander hybridisieren bzw. aneinander binden können. Mit anderen Worten weist die Funktionsnukleinsäure in ihrer Sequenz zumindest eine Base auf, welche komplementär zu zumindest einer Base der Zielnukleinsäure bzw. der zu extrahierenden Nukleinsäure ist. Vorzugsweise weist die Funktionsnukleinsäure jedoch eine größere Komplementarität zur Zielnukleinsäure auf, d.h. dass vorzugsweise die Basensequenz der Funktionsnukleinsäure nicht nur in einer Base, sondern in mehreren, vorzugsweise aufeinanderfolgenden, Basen komplementär zu einem oder mehreren Abschnitten der Basensequenz der Zielnukleinsäure ist. Mehr bevorzugt sind zumindest 5, noch mehr bevorzugt zumindest 10, viel mehr bevorzugt zumindest 15, am meisten bevorzugt zumindest 20 Basenpaare, welche besonders bevorzugt aufeinanderfolgend angeordnet sind ohne dass andere Basen dazwischenliegend angeordnet sind, komplementär zur einer entsprechenden Sequenz der Zielnukleinsäure.

Vorzugsweise sind zumindest 1%, weiter bevorzugt zumindest 5%, noch weiter bevorzugt zumindest 10%, mehr bevorzugt zumindest 20%, noch mehr bevorzugt zumindest 30%, viel mehr bevorzugt zumindest 40%, sehr viel mehr bevorzugt zumindest 50%, besonders bevorzugt zumindest 60%, ganz besonders bevorzugt zumindest 70%, am meisten bevorzugt zumindest 80% und am allermeisten bevorzugt zumindest 90% der Basensequenz der Funktionsnukleinsäure komplementär zu einer entsprechenden Sequenz der Zielnukleinsäure. Gemäß einer besonders bevorzugten Ausführungsform kann die Extraktionsnukleinsäure im Wesentlichen vollständig komplementär zu einer entsprechenden Sequenz der Zielnukleinsäure sein, d.h. dass abgesehen von wenigen Fehlstellen die Basensequenz der Funktionsnukleinsäure vollständig komplementär zu einem entsprechenden Sequenzabschnitt der Zielnukleinsäure ist. Sofern die Funktionsnukleinsäure mehrere verschiedene Abschnitte von Basensequenzen aufweist, beispielsweise einen Funktionssabschnitt, welcher zum Hybridieren an die Target-Nukleinsäure dienen soll und auch einen Verbindungsabschnitt, wie etwa einen Spacer bzw. eine Abstandshalter-Sequenz zum Beabstanden des Funktionsabschnitts von dem Heizelement, beziehen sich die oben erläuterten Ausführungen zur Komplementarität zwischen der Funktionsnukleinsäure und der Zielnukleinsäure vorzugsweise nur auf den Funktionsabschnitt der Funktionsnukleinsäure.

Ein höheres Maß an Komplementarität zwischen der Funktionsnukleinsäure und der Zielnukleinsäure, kann Vorteile hinsichtlich der Selektivität bei der Extraktion bieten. So kann ein hohes Maß an Komplementarität zwischen der Funktionsnukleinsäure und der die Zielnukleinsäure ermöglichen, dass im Wesentlichen nur die Zielnukleinsäure an die Funktionsnukleinsäure binden kann, während ein Binden anderer Nukleinsäuren aus der Probenflüssigkeit an die Funktionsnukleinsäure eine Ausnahme darstellt. Andererseits kann ein geringes Maß an Komplementarität zwischen der Funktionsnukleinsäure und der Zielnukleinsäure vorzugsweise ein Binden von unterschiedlichen Nukleinsäuren aus der Probenflüssigkeit an die Funktionsnukleinsäure ermöglichen, so dass auch andere Nukleinsäuren aus der Probenflüssigkeit extrahiert werden können und/oder die Zielnukleinsäure auch dann extrahiert werden kann, wenn die Funktionsnukleinsäure ein nur geringes Maß an Komplementarität zu dieser aufweist, beispielsweise weil die Basensequenz der Zielnukleinsäure nicht in ausreichendem Maß bekannt ist und entsprechend die Funktionsnukleinsäure nicht exakt auf die Zielnukleinsäure angepasst werden kann.

Dass das Heizelement mit einer Funktionsnukleinsäure funktionalisiert ist bedeutet dabei, dass die die Funktionsnukleinsäure an das Heizelement gebunden ist. Dies wiederum bedeutet, dass die Funktionsnukleinsäure mechanisch fest mit dem Heizelement verbunden ist, insbesondere durch eine chemische und/oder elektrostatische Bindung. Beispielsweise kann die Funktionsnukleinsäure mittels einer oder mehrerer Thiol-Bindungen und/oder Schwefelbindungen an eine Oberfläche des Heizelements gebunden sein. Vorzugsweise ist dazu das Heizelement an seiner Oberfläche zumindest teilweise mit einem Material versehen, das die Anbindung von Nukleinsäuren erlaubt. Zum Beispiel kann eine vergoldete Oberfläche genutzt werden, um die Extraktionsnukleinsäure und optional andere Nukleinsäuren über eine oder mehrere Thiol- und/oder Schwefel-Bindung(en) an das Heizelement zu binden. Auch kann zum Beispiel eine Streptavidin-Biotin-Bindung zur Anbindung der Funktionsnukleinsäure und/oder anderer Nukleinsäuren auf das Heizelement genutzt werden, wenn zum Beispiel, vorzugsweise vorher, einer der beiden Partner (Streptavidin oder Biotin) auf die Heizelemente gebunden wurde und die Funktionsnukleinsäure (vorzugsweise am 5'-Ende) mit dem anderen der beiden Partner modifiziert ist und anschließend darüber an das Heizelement gebunden wird. Auch andere Modifikationen wie zum Beispiel Amino- oder Carboxy-Gruppen können zur Bindung der Funktionsnukleinsäure auf das Heizelement genutzt werden, die Oberfläche des Heizelements kann hierfür zum Beispiel, vorzugsweise vorher, mit Epoxy und/oder einem Metall modifiziert werden. Vorzugsweise findet eine Bindung derart statt, dass das 5'-Ende der Funktionsnukleinsäure an das Heizelement gebunden ist, sodass das 3'-Ende frei ist. Dies kann insbesondere dann vorteilhaft sein, wenn die Extraktionsnukleinsäure in einer nach der Extraktion durchzuführenden Amplifikationsreaktion als ein Primer dienen soll.

Dass das Heizelement mit der Probenflüssigkeit bzw. mit der Reaktionslösung in Kontakt ist kann beinhalten, dass die Target-Nukleinsäure zumindest teilweise an die Funktionsnukleinsäure binden kann. Dies kann erfordern, dass zumindest an der Kontaktfläche zwischen dem Heizelement und der Probenflüssigkeit bzw. Reaktionslösung, also an der Oberfläche des Heizelements und/oder in der unmittelbaren Umgebung, zumindest teilweise Hybridisierungsbedingen vorherrschen, welche ein Hybridisieren der Target-Nukleinsäure mit der Funktionsnukleinsäure begünstigen und/oder ermöglichen. Beispielsweise können die Hybridisierungsbedingungen eine Temperatur und/oder einen pH-Wert und/oder eine Salzkonzentration in einem bestimmten Bereich umfassen. Gemäß einer bevorzugten Ausführungsform kann das Heizelement dabei derart beheizt werden, um in der unmittelbaren Umgebung zumindest zeitweise die erforderliche Temperatur bereitzustellen, um die Hybridisierung der Target-Nukleinsäure mit der Funktionsnukleinsäure zu ermöglichen. Gemäß einer weiteren bevorzugten Ausführungsform, kann das Heizelement und die Probenflüssigkeit vorzugsweise auch möglichst homogen beheizt werden, d.h. derart, dass die Heizelemente die gleiche oder nahezu die gleiche Temperatur aufweisen wie die Reaktionslösung, um eine effektive Hybridisierung der zu extrahierenden Nukleinsäure an die Funktionsnukleinsäure zu ermöglichen.

Dies kann vorzugsweise durch das Heizelement und/oder durch eine optionale weitere, vorzugsweise außerhalb der Probenflüssigkeit angeordneten Heizvorrichtung, realisiert werden. Hierzu kann beispielsweise die gesamte Vorrichtung, die die Heizelemente sowie die Probeflüssigkeit enthält, auf eine Basistemperatur von beispielsweise zwischen 35°C und 80° und besonders vorzugsweise zwischen 40°C und 70°C aufgeheizt werden. Eine derartige Basistemperatur kann vorzugsweise zumindest zeitweise während der Extraktion eingestellt werden. Während einer optionalen Amplifikation nach der Extraktion kann optional vom Einstellen derselben Basistemperatur abgesehen werden und/oder eine andere Basistemperatur bzw. globale Temperatur der Reaktionslösung eingestellt werden.

Eine Polymerase-Kettenreaktion bzw. PCR im Sinne der vorliegenden Erfindung ist ein Verfahren zum Vervielfältigen von Target-Nukleinsäuren, bei dem ein Vervielfältigungszyklus bestehend aus den Schritten Denaturierung, Hybridisierung und Elongation wiederholt durchlaufen wird, und zwar vorzugsweise in dieser Reihenfolge. In jedem Durchlauf kann die Anzahl der Nukleinsäuremoleküle und insbesondere der Target-Nukleinsäuren vergrößert (im typischerweise besten Fall verdoppelt) werden, sodass es zu einer exponentiellen Zunahme der Zahl von Nukleinsäuremolekülen kommen kann. Im Folgenden wird eine zu vervielfältigende Target-Nukleinsäure als "Original" bezeichnet. Das Original ist ein Einzelstrang und kann zusammen mit seinem komplementären Strang, der als "Komplement" bezeichnet wird, einen Doppelstrang bilden. Das Original und auch das Komplement können Teil einer größeren Nukleinsäure sein.

Insbesondere kann bei einer PCR eine in einem Durchlauf des Vervielfältigungszyklus entstandene Kopie des Originals eine Vorlage zum Bilden eines Komplements in einem folgenden Durchlauf und eine entstandene Kopie des Komplements eine Vorlage zur Bildung eines Originals in einem folgenden Durchlauf sein. Eine geläufige Bezeichnung für das Amplifikationsprodukt ist "Amplikon".

Der Denaturierungsschritt dient dazu, einen Nukleinsäure-Doppelstrang zu denaturieren, das heißt, ihn in seine beiden Einzelstränge aufzutrennen. So kann in dem Denaturierungsschritt zum Beispiel das Original von dem Komplement getrennt werden. Die erfindungsgemäß bevorzugte Art der Denaturierung ist eine thermische Denaturierung (auch als "Schmelzen" bezeichnet). Dazu wird zumindest ein Teil des Nukleinsäure-Doppelstrangs oder der ganze Doppelstrang einer Temperatur, als "Denaturierungstemperatur" bezeichnet, ausgesetzt, die ein Trennen der Nukleinsäure-Doppelstränge hervorruft oder zumindest begünstigt. Die bevorzugte Denaturierungstemperatur ist einerseits so hoch gewählt, dass Nukleinsäure- Doppelstränge aufgetrennt werden können. Andererseits ist die bevorzugte Denaturierungstemperatur so niedrig gewählt, dass eine DNA-Polymerase, die sich möglicherweise ebenfalls in der Probe befindet, nicht wesentlich geschädigt wird. Ein typischer Wert für die Denaturierungstemperatur ist 95° C.

Zur Erleichterung der nachfolgenden Erläuterung der Erfindung bezeichnet "Denaturierungsschritt" in der Nomenklatur der vorliegenden Erfindung den Schritt des Verfahrens, in dem die Heizeinrichtung Wärme erzeugt, um das Reaktionsvolumen zu erwärmen und dadurch eine Denaturierung doppelsträngiger Nukleinsäuremoleküle zu bewirken. Die Dauer des Denaturierungsschritts ist demnach die Summe der Zeit, in der die Heizeinrichtung in dem den Denaturierungsschritt betreffenden Durchlauf des Zyklus der PCR Wärme erzeugt. Im Fall eines Heizwiderstands als Heizeinrichtung und/oder als Heizelement ist die Dauer des Denaturierungsschritts also die Dauer einer Stromdurchleitung durch die Heizeinrichtung bzw. durch das Heizelement, um das Reaktionsvolumen zu erwärmen und dadurch eine Denaturierung doppelsträngiger Nukleinsäuremoleküle zu bewirken. Wenn die Heizeinrichtung bzw. das Heizelement in einem Durchlauf des Vervielfältigungszyklus die Wärme statt in einem in mehreren voneinander getrennten Zeitintervallen erzeugt (was, wie weiter unten erläutert, vorteilhaft sein kann), ist die Dauer des Denaturierungsschritts die Summe der Dauern dieser Intervalle. In dem so definierten Denaturierungsschritt ist insbesondere nicht eine Abgabe von Wärme aufgrund der des Heizelements eigenen Wärmekapazität eingeschlossen, und auch nicht das Abklingen der Temperatur in dem an das Heizelement angrenzenden Teil des Reaktionsvolumens, selbst wenn die dort vorhandenen Temperaturen noch innerhalb des für eine Denaturierung erforderlichen Bereichs liegen. Dies bedeutet insbesondere, dass in dem erfindungsgemäßen Verfahren auch nach dem so definierten Denaturierungsschritt noch Denaturierung stattfinden kann. Es bedeutet auch, dass die im Denaturierungsschritt abgegebene Wärme in der Regel geringer ist als die im Denaturierungsschritt erzeugte Wärme.

Das Beheizen des bzw. der Heizelemente, die vorzugsweise als resistive Heizelemente und besonders bevorzugt als Heizdrähte ausgebildet sind, kann vorzugsweise mittels kurzer elektrischer Impulse, mit welchen das bzw. die Heizelemente bestromt werden, erreicht werden. Besonders bevorzugt erfolgt dies derart, dass nur die unmittelbare Umgebung des oder der Heizelemente der Heizeinrichtung für kurze Zeit lokal erwärmt wird, vorzugsweise, um die Denaturierung der Nukleinsäuremoleküle in dem Reaktionsvolumen durchzuführen, während der Großteil des Reaktionsvolumens, d.h. der Reaktionslöung, auf einer (in diesem Sinne "globalen") Basistemperatur verbleibt, bei der insbesondere eine Elongation, vorzugsweise auch eine Hybridisierung, stattfinden kann. Dies wird vorzugsweise dadurch erreicht, dass die Dauer der Erwärmung durch die Heizeinrichtung so kurz ist, dass sich das in dem umgebenden Reaktionsvolumen entstehende Wärmefeld nur wenige Mikrometer ausbreiten kann und auf diese Weise eine Aufheizzone schafft, die vorzugsweise nur einen winzigen Bruchteil des Reaktionsvolumens umfasst. Insbesondere kann dadurch die eingebrachte Wärmemenge so gering sein, dass keine substantielle globale Erwärmung des Reaktionsvolumens stattfindet.

Die "globale Temperatur" im Sinne der vorliegenden Erfindung ist die auf den Rauminhalt bezogen durchschnittliche Temperatur des Reaktionsvolumens bzw. die Reaktionslösung, in der die PCR stattfindet, also die Temperatur, die sich nach einer Thermalisierung des Reaktionsvolumens in diesem einstellt oder einstellen würde. Die "globale Erwärmung" ist die Zunahme der so definierten globalen Temperatur.

Weiter ist mit der Erfindung erreichbar, dass sich nach dem Erwärmen, insbesondere im Denaturierungsschritt, die eingebrachte Wärme, die sich aus der Aufheizzone in den Rest des Reaktionsvolumens ausbreitet, dort nur eine vernachlässigbare globale Temperaturerhöhung hervorruft. "Vernachlässigbar" bedeutet hier insbesondere, dass die Temperaturerhöhung vorzugsweise zu gering für eine Denaturierung der Nukleinsäuremoleküle ist und besonders vorzugsweise, dass die Temperaturerhöhung zu gering ist, um die Hybridisierung und die Elongation zu stören.

Im Folgenden soll das lokale Heizen im Sinne der Erfindung noch näher erläutert werden. Durch einen Stromfluss durch ein Heizelement, welches beispielsweise als Mikro-Heizelement, wie etwa als Heizdraht mit einem Durchmesser von nur etwa 20 Mikrometer oder weniger, ausgestaltet sein kann, fängt mit Beginn des Heizpulses das Heizelement an, sich zu erwärmen. Vorzugsweise ist das Heizelement aus einem metalischen Material ausgebildet und weist eine sehr hohe Wärmeleitfähigkeit auf. Besonders bevorzugt ist das Heizelement derart ausgestaltet, dass es näherungsweise homogen über die Dauer des Heizpulses erwärmt. An der Oberfläche des Heizelements, das während der PCR in Kontakt mit der Reaktionslösung ist, wird die Wärme vom Heizelement an die Reaktionslösung übertragen, wo sie sich auf ein zunehmend größeres Volumen ausbreitet. Die Ausbreitung eines Wärmefeldes erfolgt in der Reaktionslösung durch Wärmediffussion, für die das folgende wurzelförmige Weg-Zeit-Gesetz gilt:

$$d \approx \sqrt{D \cdot t}$$

Dabei steht d für die Wegstrecke, die eine Wärmefront nach einer Zeit t entlang einer Raumrichtung in einer Reaktionslösung mit Temperaturleitfähigkeit D zurücklegt. Diese Wegstrecke d wird im Folgenden als Wärmediffusionsweite bezeichnet. Das heißt, für eine besonderes bevorzugte typische Heizdauer von beispielsweise 100μs kann die im Heizelement erzeugte Wärme in der Reaktionslösung mit einer typischen Temperaturdiffusivität (auch bekannt als Temperaturleitfähigkeit) von $D \approx 1.6 \cdot 10^{-7} m^2/s$ größenodnungsmäßig d ≈

$$\sqrt{1.6 \cdot 10^{-7} m^2/s \cdot 10^{-4} s} \approx 4\mu m$$

weit diffundieren. Mit anderen Wortenhat hat sich in diesem Zeitraum von 100μs die Wärme, die im Heizelement durch resistives Heizen erzeugt wird, in die das Heizelement umgebende Reaktionslösung in etwa 4 μm weit ausgebreitet.

Durch die räumliche Ausbreitung der Wärme entsprechend obiger Gleichung verteilt sich die vom Heizelement in die Reaktionslösung eingebrachte Wärmemenge auf ein zunehmend größeres Volumen der Reaktionsklsung, so dass sich senkrecht zur Oberfläche des Heizelement, das um eine Temperatur $\Delta T_{Local}$ (oder auch ΔT bezeichnet) heißer ist, als die globale Durchschnittstemperatur, ein mittlerer Temperaturgradient von

$\Delta T/d$ (( $\Delta T/\sqrt{D \cdot t}$ , wobei t hier die Dauer angibt) ergibt, der den Wärmetransport ermöglicht.

Dadurch können beispielsweise Temperaturgradienten erreicht werden, die vorzugsweise größer als 1K/μm, besonders vorzugsweise größer als 3K/μm und ganz besonders vorzugsweise größer als 5K/μm erreichen, um eine hohe Lokalisierung der Temperaturerhöhung zu erreichen. Alternativ oder zusätzlich sind die Wärmegradienten vorzugsweise kleiner als 1000K/μm und besonders vorzugsweise kleiner als 300K/μm. Dies kann vorteilhaft sein, um thermophoretische Effekte in der Reaktonslösung zu vermeiden.

Eine genauere Abschätzung der räumlichen Wärmeausbreitung während und nach dem Heizpuls für eine jeweilige Geometrie des Heizelements kann beispielsweise durch Finite-Elemente Methoden, wie z.B. mit kommerziellen Lösungen wie COMSOL erreicht werden, die ein numerisches Lösen der Wärmediffusionsgleichung ermöglichen.

Der Teil des Reaktionsvolumens bzw. der Reaktionslösung, in den die Wärme während des Heizpulses diffundieren kann, wird im Folgenden als "Aufheizzone" (AHZ) bezeichnet. Die Ausdehnung der Aufheizzone senkrecht zur Oberfläche des Heizelements kann näherungsweise durch die oben definierte Wärmediffusionsweite abgeschätzt werden.

Das Volumen der Reaktionslösung, das nicht in der Aufheizzone liegt, wird im Folgenden als "unbeheiztes Passiv-Volumen" bezeichnet. D.h. beispielsweise für ein zylindrisches Heizelement (z.B. ein Heizdraht) kann die AHZ als das Volumen, das sich im Abstand einer Wärmediffusionsweite d von der Zylinderoberfläche befindet (d.h. die Zylinderschale mit Dicke d), abgeschätzt werden. Wenn das Heizelement z.B. ein länglicher Zylinder mit Radius r und Länge l ist (etwa ein Draht), so kann das Volumen der Aufheizzone vorzugsweise grob abgeschätzt werden als:

$$V_{AHZ} \approx \pi \cdot l \cdot ((r + d)^2 - r^2) \,.$$

Vorzugsweise werden durch die geeignete Wahl der Heizdauer lediglich ein oder mehrere Teilvolumina der Reaktionslösung signifikant aufgeheizt, welche vorzugsweise Ausdehnungen (senkrecht von der Oberfläche des Mikroheizers gemessen), d.h. Wärmediffusionsweite von vorzugsweise $0.05\,\mu m$ bis $200\,\mu m$, vorzugsweise $0.1\,\mu m$ bis $100\,\mu m$, vorzugsweise $0.1\,\mu m$ bis $50\,\mu m$, besonders vorzugsweise $0.1\,\mu m$ bis $25\,\mu m$, besonders vorzugsweise $0.1\,\mu m$ bis $15\,\mu m$ und ganz besonders vorzugsweise $0.1\,\mu m$ bis $10\,\mu m$ aufweisen. Vorzugsweise bedeutet "signifikant erwärmt werden", dass die Temperaturerhöhung in der Entfernung einer Wärmediffusionsweite senkrecht von der Oberfläche des Heizelements vorzugsweise weniger als 50 K beträgt, mehr bevorzugt weniger als 30 K , noch mehr bevorzugt weniger als 20 K, viel mehr bevorzugt weniger als 10 K, am meisten bevorzugt weniger als 5 K beträgt. Einerseits soll durch eine ausreichende räumliche Ausdehnung des erwärmten Bereichs der Reaktionslösung senkrecht zur Oberfläche des Heizelements erreicht werden, dass die auf dem Heizelement entstehenden Amplikons, die typischerweise eine Länge von 0,02-3 $\mu m$ haben (entsprechend ca. 60-10000 Basenpaaren), möglichst homogen beheizt und somit denaturiert werden können. Zum anderen soll die Wärmediffusionsweite vorzugsweise ausreichend klein ausgebildet sein, um das Volumenverhältnis von Aufheizzone zum unbeheizten Passiv-Volumen gering zu halten.

Vorzugsweise wird die PCR derart durchgeführt, dass in mindestens einem der Durchläufe des Vervielfältigungszyklus der PCR die Heizeinrichtung bzw. das Heizelement bzw. die Heizelemente dem Reaktionsvolumen bzw. der Reaktionslösung weniger im Denaturierungsschritt erzeugte Wärme zuführen, als CR * 5° C, und wobei CR die Wärmekapazität des Reaktionsvolumens während des Erwärmens durch die Heizeinrichtung ist, und sich während des gesamten Denaturierungsschritts auf zumindest 10 % vorzugsweise jedoch auf der gesamten Kontaktfläche der Heizeinrichtung bzw. des Heizelements bzw. der Heizelemente mit dem Reaktionsvolumen kein zeitlich stabiler Temperaturgradient einstellt.

Ein Temperaturgradient gilt nach einer Dauer t1 nach dem Beginn der Erwärmung durch das Heizelement als "zeitlich stabil" im Sinne der vorliegenden Erfindung, wenn sich der Betrag seiner maximalen Steigung zu einem Zeitpunkt 2t1 um weniger als 30 % gegenüber dem Betrag seiner maximalen Steigung zum Zeitpunkt t1 geändert hat.

Dabei ist für die Feststellung der zeitlichen Stabilität lediglich der Vergleich der Beträge der maximalen Steigung relevant, nicht jedoch ob die Heizeinrichtung bzw. das Heizelement bzw. die Heizelemente zum Zeitpunkt 2t1 Wärme erzeugt oder nicht. Vorzugsweise hat sich der Betrag der Steigung zum Zeitpunkt 2t1 um weniger als 20 %, weiter bevorzugt um weniger als 15 %, mehr bevorzugt um weniger als 10 %, besonders bevorzugt um weniger als 5 % gegenüber dem Betrag seiner maximalen Steigung zum Zeitpunkt t1 geändert. Der Gradient hat seine maximale Steigung in der Regel an der Oberfläche des Heizelements bzw. der Heizelemente.

Die Denaturierung und vorzugsweise auch andere Schritte der Nukleinsäure-Vervielfältigung bzw. PCR können somit lokal in der direkten Umgebung der Heizelemente stattfinden, wobei vorzugsweise mindestens einer der benötigten Primer an der Heizeinrichtung bzw. am Heizelement bzw. an einem der Heizelemente befestigt (nachfolgend als "funktionalisiert" bezeichnet) ist, um auch das Amplikon dort entstehen zu lassen und somit eine Denaturierung bei lokaler Erwärmung zu ermöglichen. Mit anderen Worten, dadurch, dass aufgrund des Funktionalisierens der Heizeinrichtung vorzugsweise eine Lokalisierung von Schritten der PCR, insbesondere Hybridisierung, Elongation und/oder Denaturierung, sowie vorzugsweise auch die Erzeugung eines Signals zum Beobachten des Fortschritts der PCR in unmittelbarer Nähe der Heizeinrichtung erreicht wird, kann das Erwärmen des Reaktionsvolumens auf einen Bruchteil des Reaktionsvolumens beschränkt werden.

Die Kombination eines erfindungsgemäßen Verfahrens zur Extraktion einer Target-Nukleinsäure und/oder zum Vervielfältigen einer Target-Nukleinsäure kann somit in besonders vorteilhafter Weise mit einer Vorrichtung zur Durchführung einer PCR auf Basis von lokalem Heizen kombiniert werden. Ein besonderer Vorteil kann sich daraus ergeben, dass die Target-Nukleinsäure bereits mittels der Funktionsnukleinsäure(n) an das bzw. die Heizelemente gebunden ist und sich die Target-Nukleinsäure demnach bereits in der Aufheizzone befindet, welche mittels lokalem Heizen auf die Denaturierunstemperatur oder darüber hinaus erhitzt werden kann. Besonders bevorzugt sind zumindest ein Teil der Funktionsnukleinsäuren auch als Primer für die PCR ausgebildet, wodurch erreicht werden kann, dass die mittels der Funktionsnukleinsäuren an das Heizelement gebundenen Target-Nukleinsäuren bereits mit einem Primer hybridisiert sind.

Die PCR nutzt ferner vorzugsweise mindestens zwei Oligonukleotide, die als "Primer" bezeichnet werden, einen Vorwärtsprimer (auch als "Forward Primer" bezeichnet) und einen Rückwärtsprimer (auch als "Reverse Primer" bezeichnet). Der Vorwärtsprimer ist komplementär zum 3'-Ende des Originals und der Rückwärtsprimer ist komplementär zum 3'-Ende des Komplements. Im Hybridisierungsschritt (auch als "Annealingschritt" bezeichnet) hybridisiert der Vorwärtsprimer und/oder der Rückwärtsprimer an eine ihm komplementäre Sequenz im Original bzw. Komplement bzw. Amplikon. Der Hybridisierungsschritt findet gewöhnlich bei einer Temperatur statt, die ein Hybridisieren der Vorwärts- und Rückwärtsprimer an deren komplementäre Sequenzen im Original bzw. Komplement bzw. Amplikon hervorruft oder zumindest begünstigt. Sie wird vorzugsweise so gewählt, dass sie ein möglichst spezifisches Hybridisieren der Primer ermöglicht. Die Hybridisierungstemperatur liegt typischerweise zwischen 50° C und 72° C. Einer oder mehrere der Primer können vorzugsweise durch die an das Heizelement gebundene Funktionsnukleinsäure gibldet werden. Im Elongationsschritt werden die hybridisierten Primer von einem Polymerase-Enzym komplementär verlängert. So kann ausgehend von dem Vorwärtsprimer ein Komplement und ausgehend von dem Rückwärtsprimer ein Original synthetisiert werden. Zum Zweck der Elongation wird die Polymerase einer Temperatur ausgesetzt, die eine Elongation ermöglicht oder zumindest begünstigt. Bei der Verwendung einer Polymerase von dem Bakterium Thermus aquaticus (Taq) wird typischerweise eine Elongationstemperatur von 72 ° C verwendet. In manchen Ausführungsformen der PCR sind die Hybridisierungs- und die Elongationstemperaturen identisch, das heißt, beide Schritte finden bei der gleichen Temperatur statt (das heißt, es gibt nur zwei Temperaturstufen während der PCR, eine kombinierte Hybridisierungs- und Elongationstemperatur und eine Denaturierungstemperatur).

Die Denaturierungstemperatur entspricht einer Temperatur, bei welcher ein Nukleinsäure-Doppelstrang denaturiert wird, d.h. bei welcher der Nukleinsäure-Doppelstrang in seine beiden Einzelstränge aufgetrennt wird. So kann in dem Denaturierungsschritt zum Beispiel die Extraktionsnukleinsäure von der daran hybridisierten Nukleinsäure getrennt werden. Die erfindungsgemäß bevorzugte Art der Denaturierung ist eine thermische Denaturierung (auch als "Schmelzen" bezeichnet). Dazu wird zumindest ein Teil des Nukleinsäure-Doppelstrangs oder der ganze Doppelstrang einer Temperatur, welche gleich oder höher als die Denaturierungstemperatur ist, ausgesetzt, die ein Trennen der Nukleinsäure-Doppelstränge hervorruft oder zumindest begünstigt. Die bevorzugte Denaturierungstemperatur ist einerseits so hoch gewählt, dass Nukleinsäure-Doppelstränge aufgetrennt werden können. Andererseits ist die bevorzugte Denaturierungstemperatur so niedrig gewählt, dass eine DNA-Polymerase, die sich möglicherweise ebenfalls in der Reaktionslösung befindet, nicht wesentlich geschädigt wird. Ein typischer Wert für die Denaturierungstemperatur kann beispielsweise bei 95 °C liegen. Das Beheizen des Heizelements auf eine Temperatur, welche gleich oder größer der Denaturierungstemperatur ist, kann vorzugsweise den Vorteil bieten, dass sich die extrahierte Nukleinsäure zumindest teilweise von der Funktionsnukleinsäure löst und frei in die Reaktionslösung übergeht. Dies kann beispielsweise für eine anschließend durchzuführende Amplifikation der extrahierten Nukleinsäure vorteilhaft sein und/oder wenn die extrahierte Nukleinsäure von dem Heizelement entfernt bzw. getrennt werden soll.

Vorzugsweise erfolgt das Erzeugen der Relativbewegung zwischen dem Heizelement und der Probenflüssigkeit dann, wenn das Heizelement mit der Probenflüssigkeit in Kontakt ist. Alternativ oder zusätzlich erfolgt das Erzeugen der Relativbewegung zwischen dem Heizelement und der Reaktionslösung zumindest teilweise während eines Denaturierungsschrittes der Polymerase-Kettenreaktion. Dies bietet den Vorteil, dass auf diese Weise die Hybridisierung durch die Relativbewegung besonders begünstigt werden kann.

Vorzugsweise wird zumindest während des Erzeugens der Relativbewegung zwischen dem Heizelement und der Probenflüssigkeit und/oder während des Erzeugens der Relativbewegung zwischen dem Heizelement und der Reaktionslösung zumindest zeitweise eine für die Hybridisierung der Target-Nukleinsäure mit der Funktionsnukleinsäure geeignete Temperatur zumindest am Ort der Funktionsnukleinsäure bereitgestellt. Dadurch wird eine effektive Hybridisierung der Target-Nukleinsäure an die Funktionsnukleinsäure erzielt. Vorzugsweise beträgt die Temperatur zumindest 20° C, bevorzugt zumindest 30° C mehr bevorzugt zumindest 40° C, viel mehr bevorzugt zumindest 50° C und am meisten bevorzugt zumindest 60° C. Alternativ oder zusätzlich beträgt die Temperatur nicht mehr als 90° C, bevorzugt nicht mehr als 80° C, am meisten bevorzugt nicht mehr als 70° C. Dies ermöglicht eine besonders effiziente und/oder besonders spezifische Hybridisierung der Target-Nukleinsäure mit der Funktionsnukleinsäure auf den Heizelementen In einer Ausführungsform wird die Temperatur vorzugsweise so gewählt, dass eine möglichst spezifische und/oder möglichst effiziente Bindung zwischen der Target-Nukleinsäure und einer Primer-Sequenz der Funktionsnukleinsäure auf den Heizelementen erreicht wird.

Vorzugsweise erfolgt ein Bereitstellen von Wärme für das Bereitstellen der Temperatur zumindest teilweise mittels des Heizelements. Beispielsweise kann die Heizeinrichtung und/oder das Heizelement gemäß der Offenbarung in der DE 10 2016 120 124 A1 ausgebildet sein. Auf diese Weise lässt sich die Amplifikation der Target-Nukleinsäure besonders schnell durchführen.

Vorzugsweise ist die Funktionsnukleinsäure als ein Oligonukleotid ausgebildet und/oder umfasst ein solches. Besonders bevorzugt ist die Funktionsnukleinsäure als eine Extraktionsnukleinsäure ausgebildet und/oder weist eine Fänger-Nukleotidsequenz auf, welche zumindest teilweise komplementär zur Nukleotidsequenz der Target-Nukleinsäure ist und dazu geeignet ist, an die Target-Nukleinsäure zu binden. Dies begünstigt das Hybridisieren der Target-Nukleinsäure an die Funktionsnukleinsäure.

Vorzugsweise weist die Funktionsnukleinsäure eine Nukleotidsequenz auf, welche als Primersequenz für die Vervielfältigung der Target-Nukleinsäure verwendbar ist. Dies bietet den Vorteil, dass die Polymerase-Kettenreaktion in unmittelbarer Nähe zum Heizelement durchgeführt werden kann, was besonders vorteilhaft für das lokale Heizen mittels des Heizelements sein kann.

Vorzugsweise enthalten die Probenflüssigkeit und/oder die Reaktionslösung eine Reagenz oder mehrere Reagenzien, die das Hybridisieren der Target-Nukleinsäure mit der Funktionsnukleinsäure begünstigen.

Dadurch kann die Extraktion und/oder die Amplifikation verbessert werden.

Vorzugsweise erfolgt die Relativbewegung zwischen dem Heizelement und der Probeflüssigkeit und/oder der Reaktionslösung zumindest teilweise durch ein Bewegen des Heizelements. Beispielsweise kann das Bewegen des Heizelements ein Schwingen und/oder ein Vibrieren des Heizelements umfassen. Das Schwingen und/oder Vibrieren des Heizelements kann vorzugsweise mittels eines Magnetfeldes hervorgerufen werden, wobei das Magnetfeld vorzugsweise durch einen elektrischen Stromfluss im Heizelement und/oder durch ein externes Magnetfeld erzeugt wird. Beispielsweise kann dies durch elektrische Strompulse und/oder zeitlich veränderlichen Stromfluss durch die Heizelemente hervorgerufen werden, z.B. dadurch dass das Magnetfeld der stromdurchflossenen Leiter der Heizelemente miteinander wechselwirkt. Insbesondere kann die Lorentzkraft genutzt werden, um die Stromdurchflossenen Heizelemente zum Schwingen und/oder Vibrieren anzuregen. Auf diese Weise kann eine gegenseitige Anziehung und/oder Abstoßung von Teilen des oder der Heizelemente herbeigeführt werden, was zu einer elastischen Verformung von Teilen des oder der Heizelemente führen kann, sodass hierdurch die Heizelemente in Schwingungen geraten können. Die Schwingung und/oder Vibration kann vorzugsweise resonant oder nicht resonant hervorgerufen werden. Sie kann vorzugsweise dadurch verstärkt werden, dass die Heizelemente sich in einem externen Magnetfeld befinden. Die Schwingung und/oder Vibration kann vorzugsweise dadurch verstärkt werden, dass zwischen benachbarten Heizelementen unterschiedlich große Abstände herrschen.

Gemäß einer bevorzugten Ausführungsform werden die Heizelemente dadurch zum Schwingen und/oder Vibrieren angeregt, dass diese bestromt werden und derart abgeordnet sind, dass diese sich während der Bestromung gegenseitig anziehen und/oder abstoßen und auf diese Weise in Bewegung versetzt werden. Beispielsweise können eine Vielzahl von als Drähten ausgebildeten Heizelementen parallel verlaufend in einer Ebene angeordnet sein und durch das Reaktionsgefäß verlaufen. Der Abstand der Heizelemente ist dabei vorzugsweise derart gewählt, dass die gegenseitige Anziehungs- und/oder Abstoßungskraft der Heizelemente ausreichend groß ist, um die Heizelemente in eine Schwingung und/oder Virbation zu versetzen. Besonders bevorzugt sind die Heizelemente derart zueinander angeordnet, dass sich die Kräfte auf die Heizelemente während der Bestromung nicht aufhebt. Dazu können beispielsweise die mehreren Heizelemente jeweils paarweise angeordnet werden. Dies kann beispielsweise dadurch erreicht werden, dass die Abstände der Heizelemente zueinander varriiert werden. So können jeweils zwei Heizelemente in geringem Abstand zueinander parallel zueinander verlaufen, während die jeweiligen Paare von Heizelementen einen größeren Abstand zueinander aufweisen, um eine vollständige Aufhebung der Kraftwirkung auf die einzelne Heizelemente zu verhindern. Mit anderen Worten können beispielsweise die Abstände der einzelnen Heizelemente alternierend größer und kleiner ausgestaltet sein. Dies kann zu einer asymmetrisch und/oder anisotropen Krafteinwirkung auf die einzelnen Heizelemente führen, wodurch die Heizelemente zum Schwingen und/oder Vibrieren angeregt werden können.

Gemäß anderen bevorzugten Ausführungsformen können ein oder mehrere externe Magneten bereitgestellt werden, um die Kraftwirkung auf die bestromten Heizelemente zu erzielen und/oder zu verstärken und/oder zu ändern.

Alternativ oder zusätzlich kann das Schwingen und/oder Vibrieren des Heizelements zumindest teilweise durch eine Krafteinwirkung durch einen mechanischen Aktor bzw. Aktuator und/oder durch Schallwellen und/oder Ultraschallwellen hervorgerufen werden. Beispielsweise kann ein Ende des Heizelements mechanisch erregt werden, um das Schwingen und/oder Vibrieren zu verursachen. Beispielsweise ein mechanischer Aktor einen oder mehrere Piezoaktuatoren und/oder einen oder mehrere Vibrationsmotoren aufweisen, um das eine oder die mehreren Heizelemente zu, Schwingen und/oder Vibrieren zu bringen.

Gemäß einer bevorzugten Ausführungsform können beispielsweise ein oder mehrere Piezo-Aktuatoren und/oder ein oder mehrere Vibrationsmotoren verwendet werden, um das Reaktionsgefäß zum Schwingen und/oder Vibrieren anzuregen und auf diese Weise eine Relativbewegung der Flüssigkeit, d.h. der Probenflüssigkeit und/oder der Reaktionslösung, in dem jeweiligen Reaktionsgefäß relativ zu dem zumindest einen Heizelement zu erzielen. Beispielsweise kann dazu ein mit Wechselstrom beaufschlagter Piezo-Aktuator und/oder ein Vibrationsmotor (wie z.B. aus einem Handy) und/oder ein Lautsprecher verwendet werden, die durch eine geeignete mechanische Kontaktierung des Reaktionsgefäßes und/oder einer Probenplatte mit einem oder mehreren Reaktionsgefäßen diese zum Schwingen und/oder vibrieren anregen. Dies kann vorzugsweise derart erfolgen, dass entweder die Heizeinrichtung mitschwingt und/oder die Probenflüssigkeit bzw. die Reaktionslösung und Heizeinrichtung andersartig der Anregung folgen (z.B. mit anderer Amplitude und/oder Phase), so dass eine Relativbewegung entsteht.

Gemäß manchen bevorzugten Ausführungsformen wird für jedes Reaktionsgefäß ein oder mehrere eigene Aktoren, wie etwa ein oder mehrere eigene Piezo-Aktuatoren, bereitgestellt. Gemäß anderen bevorzugten Ausführungsformen kann ein Piezo-Aktuator für mehrere Reaktionsgefäße bereitgestellt werden. Gemäß wieder anderen bevorzugten Ausführungsformen können beispielsweise mehrere Aktoren bereitgestellt werden, welche in Zusammenwirkung mehrere Reaktionsgefäße und/oder die darin befindlichen Heizelemente und/oder Flüssigkeit zum Schwingen und/oder Vibrieren anregen. Gemäß einer besonders bevorzugten Ausführungsform können mehrere Reaktionsgefäße in einer Probenplatte ausgebildet sein, wobei ein oder mehrere Aktoren, wie etwa ein oder mehrere Piezo-Aktuatoren und/oder Vibrationsmotoren, bereitgestellt werden, um alle Reaktionsgefäße der Probenplatte zum Vibrieren und/oder Schwingen anzuregen. Der eine oder die mehreren Aktoren können beispielsweise seitlich und/oder unterhalb und/oder auf an der Probenplatte angeordnet sein. Besonders bevorzugt wird ein Aktor pro Reaktionskammer oder pro Probenplatte bereitgestellt.

Alternativ oder zusätzlich kann die Relativbewegung durch Bewegung der Heizelemente relativ zum Reaktionsgefäß hervorgerufen werden, z.B. indem das bzw. die Heizelemente durch das Reaktionsgefäß gezogen werden, in dem sich die Probenflüssigkeit oder die Reaktionslösung befindet und/oder durch bewegte Magnetfelder, die auf die magnetischen Heizelemente einwirken oder durch Schallwellen, die die Heizelemente zum schwingen bringen (beispielsweise im hörbaren Bereich oder auch im Ultraschallbereich).

Vorzugsweise erfolgt die Relativbewegung zwischen dem Heizelement und der Probeflüssigkeit und/oder der Reaktionslösung zumindest teilweise durch ein Bewegen der Probenflüssigkeit bzw. der Reaktionslösung.

Besonders bevorzugt erfolgt das Bewegen der Probeflüssigkeit und/oder der Reaktionslösung zumindest teilweise durch ein Pumpen der Probeflüssigkeit bzw. der Reaktionslösung in einem Reaktionsgefäß. Dies kann beispielsweise durch Pumpen der Flüssigkeit, d.h. der Probenflüssigkeit oder der Reaktionslösung durch das Reaktionsgefäß erfolgen. Das Reaktionsgefäß kann hierzu vorzugsweise eine weitere Kammer beinhalten, die den Anteil des Reaktionsvolumens aufnehmen kann, das bereits mit den Heizelementen in Kontakt gebracht wurde. Das Reaktionsvolumen kann vorzugsweise entweder einmal mit den Heizelementen in Kontakt gebracht werden oder wiederholt (z.B. durch hin und her pumpen). Das gesamte Reaktionsvolumen kann vorzugsweise größer sein, als das Volumen das gleichzeitig mit den Heizelementen in Kontakt gebracht werden kann. Das Pumpen kann z.B. dadurch erfolgen, dass ein Teil des Reaktionsgefäßes seine Geometrie verändert, also eine flexible Membran oder Fläche, die von außen bewegt wird und somit eine Bewegung der Reaktionslösung bzw. Probenflüssigkeit in der Reaktionskammer hervorruft.

Vorzugsweise erfolgt das Bewegen der Probeflüssigkeit und/oder der Reaktionslösung zumindest teilweise durch eine Beaufschlagung der Probenflüssigkeit bzw. der Reaktionslösung mit Schallwellen und/oder Ultraschallwellen. Die Beaufschlagung mit durch Ultraschall und/oder Schall kann z.B. von außerhalb der Reaktionskammer bzw. des Reaktionsgefäßes erfolgen und in die Reaktionsflüssigkeit eingekoppelt werden und dort z.B. gezielt gerichtete oder turbulente Strömungen verursachen.

Vorzugsweise erfolgt das Bewegen der Probeflüssigkeit und/oder der Reaktionslösung zumindest teilweise durch zumindest einen mechanischen Aktor. Der Aktor kann vorzugsweise extern getrieben sein. Der Aktor kann z.B. magnetische Elemente, wie Beads oder Rührfische, aufweisen, die durch ein von außen angelegtes Magnetfeld in der Reaktionsflüssigkeit bewegt werden und somit die Reaktionsflüssigkeit bewegen.

Vorzugsweise wird das Bewegen der Probeflüssigkeit und/oder der Reaktionslösung zumindest teilweise durch Konvektion hervorgerufen, z.B. durch gezieltes einbringen von Temperaturgradienten.

Vorzugsweise erfolgt die Relativbewegung zwischen dem Heizelement und der Probeflüssigkeit und/oder der Reaktionslösung zumindest teilweise durch ein Bewegen eines Reaktionsgefäßes, in dem die Probenflüssigkeit bzw. die Reaktionslösung angeordnet ist. Dies kann beispielsweise durch Schütteln, Zentrifugieren, Drehen, Vortexen, Kippen und/oder andere Bewegungsformen umfassen, die vorzugsweise dazu führen, dass sich vorzugsweise auf Grund von Trägheit oder Reibung oder ihrer Materialeigenschaften oder auf Grund anderer Effekte die Heizelemente anders bewegen als die Flüssigkeit, so dass es zu einer Relativbewegung kommt.

Selbstverständlich kann die Erzeugung der Relativbewegung auf einer Kombination mancher oder aller oben-beschriebenen Methoden zu Erzeugung der Relativbewegung basieren.

Vorzugsweise weist die Heizeinrichtung mehrere Heizelemente auf. Dadurch kann insbesondere eine größere Kontaktfläche zwischen der Probenflüssigkeit bzw. Reaktionslösung und dem Heizelement erzielt werden.

Vorzugsweise sind das eine oder die mehreren Heizelemente als elektrische und insbesondere als resistive Heizelemente ausgebildet und sind bevorzugt als Heizdraht bzw. Heizdrähte ausgebildet. Besonders bevorzugt sind die Heizelemente wie in der DE 10 2016 120 124 A1 beschrieben ausgebildet. Vorzugsweise ist das bzw. sind die Heizelemente jeweils mit einer Mehrzahl von Funktionsnukleinsäuren konjugiert, wobei die Funktionsnukleinsäuren des Heizelements gleichartig oder zumindest teilweise verschiedenartig ausgebildet sind. Besonders bevorzugt ist jedes der Heizelemente jeweils mit einer Mehrzahl von gleichartigen Funktionsnukleinsäuren konjugiert und die verschiedenen Heizelemente sind vorzugsweise mit Funktionsnukleinsäuren konjugiert, die von den Funktionsnukleinsäuren der anderen Heizelemente zumindest teilweise verschieden sind.

Heizeinrichtung

Die Heizeinrichtung kann aus einem oder mehreren Heizelementen bestehen. Alternativ kann die Heizeinrichtung zusätzlich noch weitere Elemnte aufweisen, wie etwa elektrische Zu- und/oder Ableitungen für elektrischen Strom und/oder eine Steuereinrichtung. In jedem Fall soll im Rahmen dieser Erfindung die von der Heizeinrichtung bereitgestellte Wärme sich auf die Wärme beziehen, welche mittels des Heizelements bzw. der Heizelemente in Kontakt mit der Reaktionslösung und/oder in Kontakt mit der Probenflüssigkeit bereitgestellt wird, nicht jedoch eine etwaige anderweitig entstehende Wärme, beispielsweise in oder an der Steuereinrichtung.

In einer bevorzugten Ausführung der Erfindung wird das Reaktionsvolumen bzw. die Reaktionslösung durch eine Heizeinrichtung mit einem oder mehreren Heizelementen erwärmt, wobei besonders vorzugsweise das Heizelement oder mindestens eines der Heizelemente, besonders vorzugsweise alle Heizelemente, elektrisch kontaktiert sind. Dadurch ist vorteilhafterweise erreichbar, dass das Heizelement von Strom durchflossen bzw. mit Spannung beaufschlagt werden kann. Diese bevorzugte Ausführung der Erfindung kann ausnutzen, dass elektrisch kontaktierte Heizelemente besonders einfach durch eine elektrische Steuereinrichtung betrieben und geregelt werden können. Vorzugsweise ist das oder zumindest eines der Heizelemente eine Einrichtung, die einen Stromfluss in Wärme umwandelt, zum Beispiel durch seinen Ohmschen Widerstand. Diese Ausführung der Erfindung kann ausnutzen, dass derartige Heizelemente elektrische Energie effizient in Wärme umwandeln können. Ein bevorzugtes Heizelement ist ein Heizwiderstand oder ein Peltier-Element. Bei mehreren Heizelementen könne diese seriell und/oder parallel oder teilweise seriell und teilweise parallel zueinander geschaltet sein.

Bevorzugte Heizelemente können ein Material aufweisen, das zwischen der wärmeerzeugenden Komponente des Heizelements und dem Reaktionsvolumen angeordnet ist, wie etwa eine Ummantelung bzw. eine Trennschicht. Ein solches Material, kann hilfreich sein, um das Heizelement zum Beispiel vor Korrosion oder anderweitigen chemischen Wechselwirkungen mit der PCR-Chemie zu schützen und/oder elektrisch zu isolieren (deshalb hier auch als Trennschicht bezeichnet) und kann zum Beispiel aus Polymeren bestehen. Vorzugsweise sind solche Heizelemente und das Reaktionsvolumen bzw. die Reaktionslösung maximal durch eine Trennschicht von einer Dicke von weniger als 500 $\mu$m, vorzugsweise einer Dicke von weniger als 100 $\mu$m, vorzugsweise einer Dicke von weniger als 20 $\mu$m, vorzugsweise einer Dicke von weniger als 5 $\mu$m, vorzugsweise einer Dicke von weniger als 1 $\mu$m, vorzugsweise einer Dicke von weniger als 0,2 $\mu$m, vorzugsweise einer Dicke von weniger als 0,05 $\mu$m getrennt. Ganz besonders bevorzugt ist dabei eine weitere Ausführungsform, bei der keine Trennschicht zwischen Heizelementen und der Reaktionslösung bzw. dem Reaktionsvolumen vorhanden ist oder bei der die thermischen Eigenschaften der Trennschicht so gewählt sind, dass ihre Wirkung in Bezug auf die Wärmeabgabe der Heizeinrichtung an das Reaktionsvolumen vernachlässigbar ist.

Es kann von Vorteil sein, dass möglichst überall auf dem Heizelement eine gleichmäßige Temperatur erreicht wird. Dies kann bei Heizwiderständen zum Beispiel dadurch erreicht werden, dass in jedem Teilstück oder Teilvolumen des Heizwiderstands eine gleichmäßige Stromdichte während des Heizens und ein gleichbleibendes Oberflächen-/Volumen-Verhältnis (OVV) ermöglicht wird. Vorteilhafterweise ist dies dadurch erreichbar, dass ein gleichbleibender Leiterquerschnitt und ein gleichbleibender Spannungsabfall pro Längeneinheit Leiter im gesamten Heizelement sichergestellt werden. Bei mehreren Heizelementen erwärmen diese das Reaktionsvolumen in einer bevorzugten Ausführung der Erfindung auf gleiche Weise. Die Erfindung umfasst aber auch Ausführungsformen, in denen Heizelemente das Reaktionsvolumen unterschiedlich erwärmen, zum Beispiel unterschiedlich lang oder unterschiedlich stark. Die Heizelemente der Heizeinrichtung können gleich oder unterschiedlich sein, zum Beispiel hinsichtlich ihrer Länge oder Geometrie.

Vorzugsweise wird das Reaktionsvolumen durch eine Heizeinrichtung aus einem oder mehreren Heizelementen erwärmt, wobei besonders vorzugsweise das Heizelement oder mindestens eines der Heizelemente, besonders vorzugsweise alle Heizelemente, mit dem Reaktionsvolumen in Kontakt stehen. In einer bevorzugten Ausführung der Erfindung grenzt mindestens eines der Heizelemente auf seiner gesamten Fläche an das Reaktionsvolumen bzw. die Reaktionslösung an; besonders vorzugsweise grenzen alle Heizelemente auf ihrer gesamten Fläche an das Reaktionsvolumen bzw. die Reaktionslösung an. Es ist ein erreichbarer Vorteil dieser Ausführung der Erfindung, dass das Reaktionsvolumen bzw. die Reaktionslösung von dem jeweiligen Heizelement in der Umgebung des Heizelements effizient erwärmt werden kann. Auch kann durch diese Ausführung der Erfindung vorteilhafterweise erreicht werden, dass zur Erzielung einer hohen Reaktionskinetik (speziell der Hybridisierungskinetik der Nukleinsäuremoleküle an Primer, die, wie unten ausgeführt, vorzugsweise auf dem Heizelement angeordnet sind) die Heizeinrichtung eine möglichst große zugängliche Oberfläche hat.

Es ist bevorzugt, dass die Heizelemente ein möglichst hohes OVV aufweisen, um eine möglichst effektive Abgabe der Wärme an die (unmittelbare) Umgebung zu ermöglichen, und gleichzeitig ein möglichst geringes Volumen haben, um eine geringe Wärmekapazität des Heizelements sicherzustellen. Erfindungsgemäß bevorzugte Ausführungsformen weisen ein OVV für die Heizelemente auf, das mehr als $10^3$ m$^{-1}$ (pro Meter), vorzugsweise mehr als $10^4$ m$^{-1}$ und besonders vorzugsweise mehr als $5*10^4$ m$^{-1}$. Ein zu großes OVV kann jedoch in manchen Fällen zu sehr filigranen und somit mechanisch instabilen Strukturen führen, sodass es erfindungsgemäß vorteilhaft sein kann, das OVV kleiner als $10^9$ m$^{-1}$ zu halten, vorzugsweise kleiner als $10^8$ m$^{-1}$ und in manchen Fällen sogar kleiner als $10^7$ m$^{-1}$.

Für einen langen Draht (Länge viel größer als Durchmesser) berechnet sich beispielsweise das OVV mit 2/r, wobei r der Radius des Drahtes ist. Für einen dünnen Film oder eine Folie (Dicke sehr viel kleiner als Länge und laterale Ausdehnung) berechnet sich das OVV mit 1/d, wobei d die Dicke des Films bzw. der Folie ist. Erfindungsgemäß bevorzugt ist für obige Ausführungsformen dabei lediglich die Oberfläche zu betrachten, die im Kontakt mit dem Reaktionsvolumen steht; auch ist vorzugsweise nur das Volumen zu betrachten, dessen Oberfläche(n) im Kontakt mit dem Reaktionsvolumen steht (das heißt beispielsweise, Zuleitungen, die nicht durch die Lösung verlaufen, sind erfindungsgemäß nicht als relevante Volumina und Oberflächen zu berücksichtigen). Gleiches gilt auch entsprechend für die nachfolgende Betrachtung von Volumenfüllfaktor und Wärmekapazität.

In einer bevorzugten Ausführung der Erfindung ist das Verhältnis zwischen der Oberfläche der Heizeinrichtung, die sich in Kontakt mit dem Reaktionsvolumen befindet, und dem Reaktionsvolumen größer als $0,1 \text{ m}^{-1}$, besonders vorzugsweise größer als $1 \text{ m}^{-1}$, besonders vorzugsweise größer als $5 \text{ m}^{-1}$, besonders vorzugsweise größer als $10 \text{ m}^{-1}$, besonders vorzugsweise größer als $20 \text{ m}^{-1}$, besonders vorzugsweise größer als $50 \text{ m}^{-1}$, besonders vorzugsweise größer als $100 \text{ m}^{-1}$. Mit dieser Ausführung der Erfindung kann vorteilhafterweise dadurch eine günstige Reaktionskinetik erreicht werden, dass in einem großen Teil des Reaktionsvolumens Bestandteile des Reaktionsvolumens durch Diffusion schnell die Oberfläche eines Heizelements erreichen können, um an den dort stattfindenden Schritten des Nukleinsäure-Vervielfältigungsverfahrens teilzunehmen. Auch kann in dem weiter unten beschriebenen Fall von Heizelementen, die zumindest teilweise auf ihrer Oberfläche mit einem der Reaktionspartner (zum Beispiel ein Primer) funktionalisiert sind, ausgenutzt werden, dass durch eine größere Oberfläche auch mehr Reaktionspartner zur Verfügung stehen.

Um zu verhindern, dass die Heizelement-Struktur zu filigran wird, bzw. dass die Bewegung der Nukleinsäuremoleküle und anderer im Reaktionsvolumen befindlicher Reaktanden durch zu viele Oberflächen behindert wird, ist vorzugsweise das Verhältnis der Oberfläche des Heizelements oder der Heizelemente im Verhältnis zur Größe des Reaktionsvolumens kleiner als $10^6 \text{ m}^{-1}$, besonders vorzugsweise kleiner als $10^5 \text{ m}^{-1}$, vorzugsweise kleiner als $10^4 \text{ m}^{-1}$ und ganz besonders vorzugsweise kleiner als $10^3 \text{ m}^{-1}$.

Um die dem Reaktionsvolumen von der Heizeinrichtung im Denaturierungsschritt zugeführte Wärme möglichst gering zu halten, kann es vorteilhaft sein, auch die Wärmekapazität der Heizeinrichtung bzw. des Heizelements bzw. der Heizelemente gering zu halten, da zur Erreichung einer gewissen Temperaturerhöhung auf der Oberfläche der Heizelemente eine umso größere Energiemenge benötigt wird, je größer die Wärmekapazität der Heizeinrichtung bzw. des Heizelements bzw. der Heizelemente ist. Die im Denaturierungsschritt von der Heizeinrichtung dem Reaktionsvolumen zugeführte Energiemenge breitet sich anschließend auf das gesamte Reaktionsvolumen aus. Die Wärmekapazität des Heizelements ergibt sich dabei aus dem Produkt des jeweiligen Volumens und der spezifischen volumetrischen Wärmekapazität des jeweiligen Materials, aus dem das jeweilige Volumen besteht. Ein wesentlicher Freiheitsgrad bei der Auslegung der Heizeinrichtung ist ihre Dimensionierung. Deshalb kann es vorteilhaft sein, das Volumen der Heizeinrichtung, insbesondere die Materialstärke, so gering wie möglich zu halten. Hierbei ist bemerkenswert, dass die Wärmediffusionsweite nicht von der Größe der Heizeinrichtung, sondern lediglich von der Heizdauer abhängt. In einer bevorzugten Ausführungsform der Erfindung beträgt das Volumen aller Heizelemente der Heizeinrichtung weniger als 10 %, vorzugsweise weniger als 5 %, besonders vorzugsweise weniger als 3 % und ganz besonders vorzugsweise weniger als 1 % des Reaktionsvolumens. Mit dieser Ausführung der Erfindung kann vorteilhafterweise durch einen geringen Volumenfüllfaktor eine geringe Wärmekapazität der Heizeinrichtung bzw. des Heizelements bzw. der Heizelemente erreicht werden.

In einer bevorzugten Ausführung der Erfindung umfasst die Heizeinrichtung eine Anordnung von mehreren Heizelementen, die als stromdurchflossene Leiter ausgebildet sind und von dem Reaktionsvolumen bzw. der Reaktionslösung und/oder der Probenflüssigkeit umgeben sind. Hierdurch ist vorteilhafterweise erreichbar, dass die Heizeinrichtung an vielen verschiedenen Orten des Reaktionsvolumens wirken kann. Dies ist besonders in typischen Fällen, in denen zu Beginn einer PCR nur eine sehr geringe Konzentration von zu vervielfältigenden Nukleinsäuremolekülen vorliegt und der mittlere Abstand dieser Moleküle zum nächstgelegenen Heizelement deshalb groß ist, günstig. Es lässt sich abschätzen, dass ein Nukleinsäure-Einzelstrang mit einer Länge von 100 Basenpaaren etwa eine Zeit $t = x^2/D_{DNA}$ benötigt, um sich durch Diffusion eine Strecke x von seinem Ausgangspunkt zu entfernen (wobei $D_{DNA} \approx 10^{-11} m^2/s$ beträgt). In einer bevorzugten Ausführung der Erfindung ist der räumliche Abstand eines jeden Punktes im Reaktionsvolumen zum nächstgelegenen Heizelement kleiner als 3 mm (Millimeter), vorzugsweise kleiner als 2 mm, besonders vorzugsweise kleiner als 1 mm, besonders vorzugsweise kleiner als 0,75 mm, besonders vorzugsweise kleiner als 0,5 mm und besonders vorzugsweise kleiner als 0,25 mm.

In einer Ausführungsform der Erfindung bestehen ein oder alle Heizelemente aus einem elektrisch leitfähigen Metall oder einer Metalllegierung oder einem anderen elektrisch leitfähigen Material mit niedrigem spezifischem elektrischem Widerstand, zum Beispiel Kohlenstoff, ein Halbleitermaterial oder ein leitfähiger Kunststoff.

In einer Ausführungsform bestehen ein oder alle Heizelemente nur aus einem Draht oder einem elektrischen Leiter oder aus mehreren Drähten oder elektrischen Leitern. Diese können gerade, gebogen oder zu einer Spule gewickelt sein. Mehrere Drähte oder elektrische Leiter können zueinander einen gleichen oder ungleichen Abstand haben, sich kreuzen oder nicht kreuzen. Jeder Draht oder elektrische Leiter kann einen runden, ovalen, flachen oder beliebigen anderen Querschnitt haben. Ein Heizelement kann auch einfach eine durchgängige Fläche sein, zum Beispiel eine mit Metall bedampfte flache Oberfläche.

Eine besonders bevorzugte Ausführungsform der Heizeinrichtung ist eine Anordnung, vorzugsweise eine periodische Anordnung, aus leitfähigen metallischen Drähten. Besonders vorzugsweise sind die Drähte parallel zueinander ausgerichtet. Die Drähte des Arrays haben vorzugsweise einen Durchmesser zwischen 0,5 und 100 μm (Mikrometern), besonders vorzugsweise zwischen 1 und 50 μm. Die Drähte sind vorzugsweise zwischen 20 und 1500 μm voneinander beabstandet.

Die Drähte bestehen vorzugsweise aus Gold und/oder anderen Metallen, oder sie sind als Manteldrähte ausgeführt, bei denen der Kern aus einem preiswerteren und stabileren Material, vorzugsweise einem Metall, besteht. Besonders bevorzugt sind Manteldrähte mit Edelstahl-, Molybdän- oder ganz besonders bevorzugt mit Wolframkern mit einem Mantel aus einem inerten Material, vorzugsweise Gold; solche Drähte können aufgrund der hohen Festigkeit des Kerns vorteilhafterweise sehr dünn (und somit mit hohem OW) ausgelegt werden und ermöglichen durch das Mantelmaterial trotzdem die gewünschten chemischen Eigenschaften des vorzugsweise Edelmetall-Mantels. Besonders bevorzugt sind Manteldrähte mit einem Wolframkern (Wolfram hat vorteilhafterweise eine weitaus höhere Reißfestigkeit als Gold) vorzugsweise mit einem Kerndurchmesser zwischen 5 und 40 μm und einem zwischen 0,1 und 2 μm starken Goldmantel.

In einer anderen bevorzugten Ausführungsform werden metallische Folien als Heizelement verwendet, die das Reaktionsvolumen durchziehen und die als Gitter ausgestaltet sind, etwa durch Ausstanzen, Electroforming, Laser- oder Wasserstrahlschneiden, Ätztechniken oder andere Verfahren.

In einer bevorzugten Ausführungsform der Erfindung ist das Heizelement auf ein elektrisch nicht oder nur schlecht leitendes Material aufgebracht. Bevorzugte Heizelemente bestehen aus einem metallischen Film, der auf eine nichtleitende Struktur galvanisch, chemisch, durch PVD, durch Druckverfahren oder andere Verfahren aufgebracht wurden. Dabei kann die nichtleitende Struktur zum Beispiel als sehr feines Spritzgussteil (eine bevorzugte Strukturgrößen ist kleiner als 300 μm) oder durch ein Rapid-Prototyping-Verfahren ausgeführt sein. Auch Gewebestrukturen, kommen als Heizelemente oder Träger für Heizelemente in Frage; insbesondere können derartige Materialien mit Maschenweiten zwischen 20 μm und 3 mm, besonders vorzugsweise zwischen 100 μm und 1,5 mm als Heizelement oder als Träger für Heizelemente dienen. Sind die Gewebestrukturen selbst leitfähig, dann können sie vorteilhafterweise als Ganzes als Heizelement dienen; sind sie aus nicht leitendem Material (zum Beispiel einem Kunststoff) können sie metallisiert werden, sodass der Strom nur durch eine dünne Oberflächenschicht (typischerweise < 10 μm) fließt, sodass sich ein große Oberfläche ergibt. Mit anderen Worten, die Drähte oder Fasern des Gewebes oder Gitters haben eine vergleichsweise große Oberfläche, aber aktiv beheizt wird nur das dünne, aufgebrachte metallische Volumen. Hierbei ist im Sinne der Erfindung nur der Teil als Heizelement anzusehen, durch den im Wesentlichen der Strom fließt; wird zum Beispiel eine Kunststoff-Struktur aus PMMA mit einem Goldfilm bedampft, dann ist nur der Goldfilm als Heizelement anzusehen. Funktionalisieren der Heizeinrichtung

In einer bevorzugten Ausführung der Erfindung ist mindestens eines der Heizelemente der Heizeinrichtung mit einer oder mehreren Funktionsnukleinsäuren konjugiert, das heißt, Funktionsnukleinsäuren sind mit dem Heizelement verbunden. Besonders vorzugsweise sind alle Heizelemente der Heizeinrichtung mit Funktionsnukleinsäuren konjugiert. Damit kann vorteilhafterweise erreicht werden, dass die Funktionsnukleinsäuren durch die Heizeinrichtung spezifisch erwärmt werden, ohne dass das ganze Reaktionsvolumen erwärmt werden muss. Sind mehrere Heizelmente in einem Reaktionsvolumen enthalten, können die Heizelemente vorzugsweise mit gleichen oder verschiedenen Funktionsnukleinsäuren konjugiert sein.

Gemäß einer bevorzugten Ausführungsform sind zumindest manche der Funktionsnukleinsäuren als Extraktionsnukleinsäuren ausgebildet. Dies bietet den Vorteil, dass die Funktionsnukleinsäuren mit in der Probenflüssigkeit befindlichen Target-Nukleinsäuren hybridisieren können und auf diese Weise die Target-Nukleinsäuren an das Heizelement binden können. Durch ein Entfernen des Heizelements von der Probenflüssigkeit können die gebundenen Target-Nukleinsäuren sodann aus der Probenflüssigkeit extrahiert werden.

In einer besonders bevorzugten Ausführung der Erfindung ist das oder sind die Heizelemente mit Funktionsnukleinsäuren konjugiert, die als Primer für eine PCR ausgestaltet sind, wobei die Primer ganz besonders vorzugsweise als Vorwärts- und/oder Rückwärtsprimern des PCR-Verfahrens ausgebildet sind. Dabei können die Funktionsnukleinsäuren sowohl als Extraktionsnukleinsäuren und als auch als Primer ausbgebildet sein. Alternativ oder zusätzlich können auch manche Funktionsnukleinsäuren als Extraktionsnukleinsäure ausgebildet sein, während andere Funktionsnukleinsäuren als Primer für eine PCR in der Reaktionslösung ausgebildet sind.

In einer bevorzugten Ausführung der Erfindung sind auf einem Heizelement oder einem Abschnitt eines Heizelements Vorwärtsprimer, jedoch keine Rückwärtsprimer und/oder auf einem Heizelement oder einem Abschnitt eines Heizelements Rückwärtsprimer, jedoch keine Vorwärtsprimer angebracht. Die Moleküle des jeweils anderen Primers können frei im Reaktionsvolumen suspendiert sein. Sofern eine Trennschicht zwischen wärmeerzeugender Komponente der Heizeinrichtung und dem Reaktionsvolumen verwendet wird, muss die Funktionalisierung so erfolgen, dass die Funktionsnukleinsäuren aus der Reaktionslösung bzw. auas der Probenflüssigkeit zugänglich sind, das heißt, dass sie bevorzugt auf der Oberfläche des Heizelements angebracht sind.

In einer weiteren bevorzugten Ausführung ist mindestens eines der Heizelemente sowohl mit Vorwärts- als auch Rückwärtsprimern konjugiert. In einer besonders bevorzugten Ausführung sind alle Heizelemente der Heizeinrichtung sowohl mit Vorwärts- als auch Rückwärtsprimern konjugiert. Mit dieser Ausführung der Erfindung kann mit Vorteil erreicht werden, dass das PCR-Produkt eines Vorwärtsprimers zum Hybridisieren mit dem Rückwärtsprimer desselben Heizelements nur einen kurzen Weg zurücklegen muss, sodass eine Hybridisierung schneller stattfinden und deshalb das PCR-Verfahren schneller durchlaufen werden kann.

In einer bevorzugten Ausführung der Erfindung wird ein Heizelement an seiner Oberfläche mit einem Material versehen, das die Anbindung von Nukleinsäuren erlaubt. Zum Beispiel kann eine vergoldete Oberfläche genutzt werden, um einen Primer über eine oder mehrere Thiol-Bindung(en) auf ein Heizelement zu binden. Auch kann zum Beispiel eine Streptavidin-Biotin-Bindung zur Anbindung eines Primers auf die Heizelemente genutzt werden, wenn zum Beispiel, vorzugsweise vorher, einer der beiden Partner (Streptavidin oder Biotin) auf die Heizelemente gebunden wurde und der Primer (am 5'-Ende) mit dem anderen der beiden Partner modifiziert ist und anschließend darüber an das Heizelement gebunden wird. Auch andere Modifikationen wie zum Beispiel Amino- oder Carboxy-Gruppen können zur Bindung von Primern auf die Heizelemente genutzt werden, die Oberfläche des Heizelements kann hierfür zum Beispiel, vorzugsweise vorher, mit Epoxy modifiziert werden. Vorzugsweise findet eine Bindung derart statt, dass das 5'-Ende des Primers an die Heizelemente gebunden ist, sodass das 3'-Ende frei ist und somit während der PCR durch die Polymerase elongiert werden kann.

In der Ausführungsform, in der sowohl Vorwärts- als auch Rückwärtsprimer auf einem Heizelement immobilisiert sind, kann der Abstand zwischen den Primermolekülen unterschiedlicher Art bewusst so gewählt werden, dass sie durchschnittlich einen Abstand von zum Beispiel weniger als 1 nm (Nanometer), weniger als 3 nm, weniger als 5 nm, weniger als 15 nm oder weniger als 50 nm zueinander aufweisen. Mit dieser Ausführungsform lässt sich vorteilhafterweise ausnutzen, dass sobald ein Vorwärtsprimer auf der Oberfläche des Heizelements elongiert wurde, dieser neu geschriebene Nukleinsäurestrang nach Denaturierung mit einem entsprechenden benachbarten Rückwärtsprimermolekül auf der Oberfläche oder mit einem Rückwärtsprimermolekül in der Nähe hybridisiert. Da dieser Prozess auf der Oberfläche der Heizelemente stattfindet, sind die lokalen Konzentrationen extrem hoch; so muss der neue Nukleinsäurestrang nicht mehrere Mikrometer durch Diffusion zurücklegen, um ein Rückwärtsprimermolekül zu finden, weil in der unmittelbaren Umgebung - nur wenige Nanometer entfernt - viele Reaktionspartner vorhanden sind (gleiches gilt umgekehrt mit elongierten Rückwärtsprimermolekülen, die immobilisierte Vorwärtsprimermoleküle in der Umgebung finden).

In einer Ausführungsform befinden sich neben mindestens der oder den Funktionsnukleinsäuren auch Fülloligonukleotide und/oder Füllmoleküle auf den Heizelementen, das heißt Oligonukleotide oder Moleküle, die nicht aktiv als Primer und/oder als Extraktionsnukleinsäure und/oder Fluoreszenz-Sonde oder Target-Nukleinsäure an der PCR teilnehmen, sondern lediglich zur Oberflächenabsättigung oder -passivierung dienen, um ein unspezifisches (also nicht durch gezielte Hybridisierung mit Primern) Binden eines Amplikons oder einer Target-Nukleinsäure auf die Oberfläche des Heizelements zu verhindern. Fülloligonukleotide haben vorzugsweise eine Länge zwischen 5 und 50 Nukleotiden, besonders vorzugsweise zwischen 10 und 40 Nukleotiden und ganz besonders vorzugsweise zwischen 20 und 30 Nukleotiden. Sie können insbesondere auch nur aus einer Nukleotidart bestehen, zum Beispiel A30-Sequenzen mit 30 Adenin-Basen. Füllmoleküle können zum Beispiel Biotin oder Polyethylenglycol sein, zum Beispiel zusätzlich mit einer funktionalen Gruppe wie zum Beispiel Thiol versehen, um die Füllmoleküle auf der Heizelement-Oberfläche zu immobilisieren. Füllmoleküle können aber auch zum Beispiel Bovines Serumalbumin sein.

In einer weiteren bevorzugten Ausführung des Verfahrens weisen die Oligonukleotide und/oder Funktionsnukleinsäuren auf den Heizelementen als Teilsequenz eine Spacersequenz auf. Die Spacersequenz liegt dabei auf der dem Heizelement zugewandten Seite des jeweiligen Oligonukleotids und/oder der Funktionsnukleinsäure. So dient die Spacersequenz dem übrigen Teil des Oligonukleotids als Abstandhalter. In einer bevorzugten Ausführungsform enthält ein Oligonukleotid sowohl eine Teilsequenz, die die Funktion eines Primers hat und als Primersequenz bezeichnet wird, als auch eine Teilsequenz, die eine Spacersequenz ist. Dadurch, dass die Primersequenzen durch die Spacersequenzen von den Heizelementen weiter beabstandet sind, können vorteilhafterweise die zu vervielfältigenden Nukleinsäuren und die DNA-Polymerasen einen besseren Zugang zu den Primersequenzen finden.

In einer bevorzugten Ausführungsform des erfindungsgemäßen Verfahrens befinden sich zwischen Spacersequenz und Primersequenz eine oder mehrere nicht-basische (abasische) Modifikationen, die das Überschreiben der Spacersequenz durch die Polymerase verhindern; der diesbezügliche Inhalt der Patentanmeldung DE 102013215168 A1 ist durch Verweis Teil der vorliegenden Offenbarung. Derartige Modifikationen können zum Beispiel 1',2'-Dideoxyribose (dSpacer), Triethylene-Glycol-Spacer (Spacer 9) oder Hexa-Ethyleneglycol-Spacer (Spacer 18) sein, die die weitere Polymerase-Aktivität in 3'-Richtung verhindern. Hierdurch kann erreicht werden, dass die Spacersequenz nicht als Vorlage für den durch die Polymerase synthetisierten Nukleinsäurestrang dient und das entstehende PCR-Produkt nicht unnötig lang wird. Ein verlängertes PCR-Produkt, das auch die komplementäre Sequenz zur Spacersequenz enthält, hätte eine deutlich erhöhte Schmelztemperatur mit den Oligonukleotiden auf den Nanopartikeln und würde in folgenden Hybridisierungsschritten unnötig unspezifisch hybridisieren und somit die gesamte PCR unspezifischer machen.

Dauer der Erwärmung

Vorzugsweise variiert die Wärmezuführung durch die Heizeinrichtung während der PCR. Besonders vorzugsweise variiert die Wärmezuführung durch die Heizeinrichtung während wenigstens eines Durchlaufs des Vervielfältigungszyklus der PCR, besonders vorzugsweise während mindestens drei, besonders vorzugsweise in mindestens 10, besonders vorzugsweise in mindestens 20, der Durchläufe des Vervielfältigungszyklus der PCR, besonders vorzugsweise periodisch.

Die Dauer der Erwärmung durch die Heizeinrichtung im Denaturierungsschritt (im Folgenden wird die Erwärmung durch die Heizeinrichtung im Denaturierungsschritt auch als "Heizimpuls" bezeichnet) liegt in einer bevorzugten Ausführung der Erfindung bei mindestens einem, vorzugsweise bei mindestens drei, besonders vorzugsweise bei mindestens 10, besonders vorzugsweise bei mindestens 20, der Durchläufe des Vervielfältigungszyklus der PCR im Intervall zwischen 100 ns (Nanosekunden) und 30 ms, besonders vorzugsweise zwischen 0,5 µs (Mikrosekunden) und 10 ms, besonders vorzugsweise im Intervall zwischen 1 µs und 5 ms, besonders vorzugsweise zwischen 1 µs und 3 ms, besonders vorzugsweise zwischen 1 µs und 1 ms, besonders vorzugsweise zwischen 1 µs und 800 µs und ganz besonders vorzugsweise im Intervall zwischen 1 µs und 500 µs. Es ist ein erreichbarer Vorteil dieser Ausführung der Erfindung, dass eine Lokalisierung der Wärme und somit der resultierenden Temperaturverteilung erreicht werden kann; mit anderen Worten, durch die kurze Heizdauer wird wenig Wärme vom Heizelement durch Wärmediffusion in die Lösung transportiert. Gleichzeitig ist mit dieser Ausführung der Erfindung erreichbar, dass die Heizdauer nicht zu kurz ist, um während der Zeit der lokalen Erwärmung ein ausreichendes Aufschmelzen bzw. Entwinden der Nukleinsäuredoppelstänge zu erlauben und/oder um zu ermöglichen, dass sich während der Zeit der lokalen Erwärmung die beiden Einzelstränge durch Brownsche Bewegung (und oder andere Kräfte) weit genug voneinander entfernen können, um nicht wieder miteinander zu rehybridisieren.

In einer bevorzugten Ausführung der Erfindung macht die Dauer des Denaturierungsschritts nur einen geringen Bruchteil der Gesamtdauer der PCR aus. Vorzugsweise macht der Denaturierungsschritt während wenigstens eines Durchlaufs des Vervielfältigungszyklus der PCR, besonders vorzugsweise während mindestens drei, besonders vorzugsweise während mindestens 10, besonders vorzugsweise während mindestens 20 der Durchläufe des Vervielfältigungszyklus der PCR weniger als 10 %, weiterhin vorzugsweise weniger als 5 %, besonders vorzugsweise weniger als 3 %, besonders vorzugsweise bzw. weniger als 1 %, besonders vorzugsweise weniger als 0,5 %, besonders vorzugsweise weniger als 0,05 % und besonders bevorzug weniger als 0,01 % der Zeit aus, die für den jeweiligen gesamten Durchlauf des Vervielfältigungszyklus der PCR verwendet wird. Durch diese Ausführung der Erfindung ist vorteilhafterweise erreichbar, dass praktisch während der gesamten Dauer der PCR Hybridisierungen stattfinden können. Da die Polymerase in der lokalen PCR praktisch während der gesamten Zeit arbeiten kann, lässt sich die Prozesszeit verkürzen. Es ist auch erreichbar, dass aufgrund der nur lokal erfolgten, und zudem sehr kurzen Erwärmung die beteiligten Polymerase-Enzyme und auch andere Reaktionspartner geschont werden und weniger schnell ihre Prozessivität verlieren.

In einer bevorzugten Ausführung der Erfindung ist während wenigstens eines Durchlaufs des Vervielfältigungszyklus der PCR, besonders vorzugsweise während mindestens drei, besonders vorzugsweise während mindestens 10, besonders vorzugsweise während mindestens 20, der Durchläufe des Vervielfältigungszyklus der PCR die Dauer des Denaturierungsschritts $t_{heat}$ kür-zer als $t_{heat} \leq (s1 \cdot |x|)^2/D)$, wobei s1 ein Skalierungsfaktor, $|x|$ der kritische Abstand und D die Temperaturleitfähigkeit sind. Der Skalierungsfaktor s1 beträgt vorzugsweise s1 = 100, besonders vorzugsweise s1 = 10, besonders vorzugsweise s1 = 1, besonders vorzugsweise s1 = 0,1, besonders vorzugsweise s1 = 0,01. Der kritische Abstand $|x|$ ist der minimale Abstand von einem Punkt auf der Heizeinrichtung bzw. dem Heizelement zu dem jeweils nächstgelegenen Punkt auf einem nicht direkt angrenzenden Teil der Heizeinrichtung, zum Beispiel zum nächstgelegenen Heizelement der Heizeinrichtung. Falls parallele Drähte als Heizeinrichtung verwendet werden, ist beispielsweise der Abstand zwischen zwei Drähten der kritische Abstand. Falls der oder die Heizelemente aus einer 2D-Struktur aufgebaut sind (etwa ein Gitter, Gewebe, Waben, etc.), ist die Maschenweite bzw. die Größe der Bohrungen/Aussparungen die maßgebliche Größe $|x|$; falls der oder die Heizelemente aus einer 3D-Struktur bestehen, ist die mittlere Porengröße die maßgebliche Größe $|x|$.

In einer alternativen bevorzugten Ausführungsform kann der kritische Abstand periodisch aufgebauter Heizeinrichtungen mittels Fourier-Transformation der räumlichen Struktur der Heizeinrichtung (beispielsweise mittels numerischer Methode basierend auf einem 3D CAD-Modell der Heizeinrichtung) ermittelt werden. Hierzu wird der Kehrwert der Ortsraumfrequenz mit dem betragsmäßig höchstem Beitrag gebildet, der gleich dem kritischen Abstand ist. (Die Fouriertransformation kann hierzu 3D durchgeführt werden, oder 1D bzw. 2D entlang verschiedener Raumachsen).

Mit dieser Ausführung der Erfindung ist erreichbar, dass die Heizdauer so kurz ist, dass die Wärmediffusionsweite viel kleiner ist als der mittlere Abstand |x|, sich Wärmefelder von benachbarten Heizelementen oder allgemein benachbarten, nicht angrenzenden Teilen der Heizeinrichtung also nicht überlappen. Insbesondere können Skalierungsfaktoren größer als 1 vorteilhaft sein für sehr lange Amplikons, bei denen die Entwindung der beiden Nukleinsäurestränge länger dauert (die Dauer bis sich ein Nukleinsäure-Doppelstrang durch Brownsche Bewegung entwinden kann nimmt mit der vierten Potenz der Länge zu); Skalierungsfaktoren unter 1 können für eine bestmögliche Heiz- und Kühl-Dynamik vorteilhaft sein.

Elektrizitätsspeicher

Eine bevorzugte erfindungsgemäße Vorrichtung ist so ausgelegt, dass ihre elektrische Leistungsaufnahme zu keinem Zeitpunkt während der PCR 50 W, besonders vorzugsweise 20 W, besonders vorzugsweise 10 W, besonders vorzugsweise 3 W, besonders vorzugsweise 2,5 W, besonders vorzugsweise 1,5 W, besonders vorzugsweise 0,5 W überschreitet. Diese Einschränkung gilt nicht für eventuell höhere Leistungsaufnahmen während des Einschaltvorgangs der Vorrichtung, wie sie durch technisch bedingte Einschaltströme verursacht werden können. Solche erhöhten Leistungsaufnahmen gelten nicht als Leistungsaufnahmen während der PCR, werden hier also nicht berücksichtigt. Mit dieser Ausführung der Erfindung ist vorteilhafterweise erreichbar, dass die Vorrichtung an üblichen portablen Stromquellen betrieben werden kann, zum Beispiel an einer Kraftfahrzeugbatterie, an dem Zigarettenanzünder eines Kraftfahrzeugs oder an einem Anschluss eines PC, eines Tablet-Computer oder eines Mobiltelefons, zum Beispiel an einem USB- oder einem Apple Lightning-Anschluss.

Vorzugsweise umfasst die Vorrichtung einen Elektrizitätsspeicher. Sie ist vorzugsweise so eingerichtet, dass die in dem Elektrizitätsspeicher vorgehaltene elektrische Energie im Verhältnis zum Fassungsvermögen des Reaktionsgefäßes größer als 0,1 J/mL, besonders vorzugsweise größer als 1 J/mL, besonders vorzugsweise größer als 2 J/mL, besonders vorzugsweise größer als 3 J/mL ist. Mit dem Elektrizitätsspeicher kann vorteilhafterweise die Elektrizität für den aufgrund variierender Wärmezuführung auch variierende Elektrizitätsbedarf der Heizeinrichtung zwischengespeichert werden.

Eine einen Elektrizitätsspeicher umfassende erfindungsgemäße Vorrichtung ist vorzugsweise so eingerichtet, dass die in dem Elektrizitätsspeicher vorgehaltene elektrische Energie im Verhältnis zum Fassungsvermögen des Reaktionsgefäßes kleiner als 100 J/mL, besonders vorzugsweise kleiner als 50 J/mL, besonders vorzugsweise kleiner als 30 J/mL ist. Diese Ausführung der Erfindung kann ausnutzen, dass der Elektrizitätsspeicher wegen einer besonders effizienten Erwärmung durch die Heizeinrichtung klein ausgelegt werden kann. Dadurch kann die erfindungsgemäße Vorrichtung mit Vorteil besonders kompakt, preiswert und portable ausgeführt werden.

Ein bevorzugter Elektrizitätsspeicher umfasst einen oder mehrere Kondensator(en), Spule(n) oder Batterie(n) oder eine Kombination der vorgenannten. In einer bevorzugten Ausführung der Erfindung ist die Speicherkapazität des Energiespeichers so ausgelegt, dass er mindestens 20 %, besonders vorzugsweise mindestens 40 %, besonders vorzugsweise mindestens 50 %, besonders vorzugsweise mindestens 60 %, besonders vorzugsweise mindestens 80 %, besonders vorzugsweise mindestens 100 %, besonders vorzugsweise mindestens 150 %, besonders vorzugsweise mindestens 200 %, besonders vorzugsweise mindestens 300 % der elektrischen Energie vorhalten kann, die für den Denaturierungsschritt eines Durchlaufs des Vervielfältigungszyklus der PCR benötigt wird. Mit dieser Ausführung der Erfindung kann vermieden werden, dass eine Stromquelle zur Verfügung gestellt werden muss, die über die gesamte Dauer des Denaturierungsschritts die für den Denaturierungsschritt erforderliche elektrische Leistung zur Verfügung stellen kann. Stattdessen kann vorteilhafterweise in der Zeit zwischen den Denaturierungsschritten, die in der Regel wesentlich länger ist als die Dauer des Denaturierungsschritts, der Energiespeicher aufgeladen werden. Auf diese Weise ist erreichbar, dass die erfindungsgemäße Vorrichtung mit einer auf ihre elektrische Leistung bezogenen schwächeren Stromquelle, zum Beispiel mit einem schwächeren Netzgerät, ausgestattet werden kann. Sofern der Elektrizitätsspeicher weniger als 100 % der für den Denaturierungsschritt eines Durchlaufs des Vervielfältigungszyklus der PCR benötigten Energie zur Verfügung stellt, kann er vorteilhafterweise sehr klein gehalten werden, jedoch muss dann die restliche Energie von einer zusätzlichen, entsprechend dimensionierten Stromquelle, zum Beispiel einem Stromnetzanschluss, während des Denaturierungsschrittes bereitgestellt werden. Werte von 100 % und mehr sind vorteilhaft, da dann die Stromquelle, die den oder die Kondensatoren speist, entsprechend klein gehalten werden kann und zudem die Spannung nicht durch die Belastung des Heizens einbricht; insbesondere ist vorteilhafterweise erreichbar, dass eine Stromquelle der erfindungsgemäßen Vorrichtung lediglich so dimensioniert sein muss, dass sie in der Lage ist, über die Dauer eines Vervielfältigungszyklus der PCR, nicht aber schon während der weitaus kürzeren Denaturierungszeit, die Energiemenge, die für den Denaturierungsschritt benötigt wird, bereitzustellen.

In einer bevorzugten Ausführung der Erfindung überschreitet in mindestens einem, vorzugsweise in mindestens drei, besonders vorzugsweise in mindestens 10, besonders vorzugsweise in mindestens 20, der Durchläufe des Vervielfältigungszyklus der PCR das Verhältnis zwischen der elektrischen Leistungsaufnahme der Vorrichtung und dem Fassungsvermögen des Reaktionsgefäßes zu keinem Zeitpunkt während der PCR 1 W/mL, vorzugsweise 0,5 W/mL, besonders vorzugsweise 0,25 W/mL, besonders vorzugsweise 0,1 W/mL. Diese Einschränkung gilt nicht für eventuell höhere Leistungsaufnahmen während des Einschaltvorgangs der Vorrichtung, wie sie durch technisch bedingte Einschaltströme verursacht werden können. Solche erhöhten Leistungsaufnahmen gelten nicht als Leistungsaufnahmen während der PCR, werden hier also nicht berücksichtigt.

In einer bevorzugten Ausführung der Erfindung ist der Elektrizitätsspeicher so ausgelegt, dass er die Energie für die Denaturierungsschritte von mindestens 5, besonders vorzugsweise mindestens 10, besonders vorzugsweise mindestens 20, besonders vorzugsweise mindestens 40, besonders vorzugsweise mindestens 100 Durchläufen des Vervielfältigungszyklus einer PCT vorhalten kann. Dadurch kann vorteilhafterweise eine Vorrichtung, insbesondere eine tragbare Vorrichtung, geschaffen werden, die unabhängig von einer anderen Stromquelle eine oder sogar mehrere vollständige Polymerase-Kettenreaktionen durchführen kann.

Bevorzugte Kondensatoren sind hochkapazitive Kondensatoren, vorzugsweise Elektrolytkondensatoren oder Super-Caps, besonders vorzugsweise jeweils mit geringem ESR-Wert. Solche Kondensatoren sind preiswert auf dem Markt erhältlich und einfach zu dimensionieren. Zum Beispiel kann anhand der Gleichung

$$ Q = \frac{1}{2}CU^2 \quad \text{bzw.} \quad C = \frac{2 \cdot Q}{U^2} $$

berechnet werden, dass zur Bereitstellung von 1 J (Joule) elektrischer Energie (was bei nahezu verlustloser Umwandlung der elektrischen Energie in Wärme gemäß einer Ausführungsform für den Denaturierungsschritt eines Reaktionsvolumens der Größe 1 mL genügen würde) bei einer Spannung U von 30 V ein Kondensator mit einer Kapazität von 2222 μF (Mikrofarad) genügen würde. Allerdings wäre der Kondensator dann am Ende des Heizimpulses vollständig entladen, sodass in der Praxis die Verwendung eines Kondensators mit mindestens 1,5-mal höherer Kapazität empfehlenswert ist, wenn nicht die Stromquelle einen erheblichen Teil des Stroms liefern kann.

Bei einem bevorzugten erfindungsgemäßen Energiespeicher ist die Kapazität des Kondensators oder, im Falle mehrerer Kondensatoren, die Summe der Kapazitäten dieser mehreren Kondensatoren größer als 100 μF, besonders vorzugsweise größer als 200 μF, besonders vorzugsweise größer als 500 μF, besonders vorzugsweise größer als 1 mF (Millifarad), besonders vorzugsweise größer als 1,5 mF. Bei einem bevorzugten erfindungsgemäßen Energiespeicher ist das Verhältnis zwischen der Kapazität des Kondensators oder, im Falle mehrerer Kondensatoren, die Summe der Kapazitäten dieser mehreren Kondensatoren und der Größe des Reaktionsvolumens größer als 0,01 mF/mL (Millifarad pro Milliliter), besonders vorzugsweise größer als 0,1 mF/mL, besonders vorzugsweise größer als 1 mF/μL, besonders vorzugsweise größer als 5 mF/mL, besonders vorzugsweise größer als 10 mF/mL. Mit dieser Ausführung der Erfindung kann vorteilhafterweise genug Energie zwischengespeichert werden, um im Denaturierungsschritt eine ausreichende Erwärmung des Heizelements zu erreichen.

Besonders bevorzugte Batterien sind hochstromfähige Batterien oder Akkumulatoren, insbesondere Lithium-Polymer-Akkumulatoren, Lithium-Ionen oder Lithium-Eisenphosphat-Akkumulatoren. In einer Ausführung der Erfindung wird oder werden die Batterie(n) in Kombination mit einem oder mehreren Kondensatoren eingesetzt. In einer anderen Ausführung wird oder werden die Batterie(n), besonders vorzugsweise Lithium-Eisenphosphat-Akkumulatoren, die sich durch einen vorteilhaft geringen Innenwiderstand auszeichnen, ohne zusätzliche Verwendung eines Kondensators eingesetzt. Es kann erfindungsgemäß günstig sein, wenn die Zuleitungen zwischen Kondensator(en) und/oder Batterien bis zum Heizelement eine möglichst kurze Länge aufweisen, um störende Induktivitäten und ohmsche Widerstände der Zuleitungen zu reduzieren.

Reaktionsgefäß

Eine bevorzugte Vorrichtung zum Vervielfältigen von Nukleinsäuren weist zumindest ein Reaktionsgefäß zur Aufnahme des Reaktionsvolumens bzw. der Reaktionslösung und/oder der Probenflüssigkeit auf. Als Reaktionsgefäß des erfindungsgemäßen Verfahrens können klassische PCR-Reaktionsgefäße wie PCR-Röhrchen (auch als PCR-Tubes bezeichnet) oder Verbünde von PCR-Tubes (wie zum Beispiel sogenannte 8er-Stripes) oder Multiwellplatten dienen, aber auch zum Beispiel flache Platten oder andere Formen, die befüllt werden können. Die Heizeinrichtung bzw. das Heizelement oder die Heizelemente können bereits während des Produktionsprozesses (zum Beispiel Spritzguss) in die Reaktionsgefäße eingebracht werden (zum Beispiel Drähte, die die Wände von Multiwellplatten oder PCR-Tubes durchdringen) oder nach dem Produktionsprozess hinzugefügt werden (wie zum Beispiel Drähte in Form von Spulen, die in die einzelnen Wells einer Multiwellplatte gehängt werden können).

Die Heizeinrichtung wird vorzugsweise in einen Probenträger und/oder eine Probenplatte eingebracht, der die Heizeinrichtung und das Reaktionsvolumen der Art aufnehmen kann, sodass die Heizeinrichtung besonders gut zugänglich für Target-Nukleinsäuren in der Probenflüssigkeit und/oder der Reaktionslösung ist. Der Probenträger und/oder die Probenplatte ermöglicht insbesondere eine elektrische Kontaktierung der Heizeinrchtung und ermöglicht eine dichte und auslaufsichere Aufnahme der Probenflüssigkeit und/oder der Reaktionslösung. Hierzu ist vorzugsweise ein Teil der Heizeinrichtung und/oder der Zuleitungen zur Heizeinrichtung in den Probenträger bzw. in die Probenplatte eingelassen, z.B. durch umspritzen ("over-moulding") der Heizeinrichtung in einem Spritzgussprozess oder durch einen schichtweise verklebten Aufbau wie in der DE 10 2016 120 124 B beschrieben. Insbesondere kann die erfindungsgemäße Heizeinrichtung vorzugsweise auch in eine mikro- oder makrofluidische Kartusche integriert werden (z.B. für eine Vor-Ort-Analytik), die durch die erfindungsgemäßen Verfahren ggf. in Kombination mit konventionellen Verfahren eine teilweise oder vollständige Integration eines molekulardiagnostischen oder DNA-analytischen Detektionsprozesses ermöglicht. In einer besonders präferierten Ausführungsform kann z.B. die Relativbewegung zwischen Reaktionslösung bzw. Probenflüssigkeit und Heizeinrichtung auf einer teil- oder vollintegrierten Kartusche dadurch realisiert werden, dass die Reaktionslösung auf der Kartusche vor und zurück bewegt wird (z.B. durch Pumpen), was z.B. dadurch erreicht werden kann, dass ein Bellow und/oder Blister auf einer Kartusche durch eine externe Aktorik betätigt wird und die Flüssigkeit sich in den Reaktionsgefäßen/-volumen auf diese Weise bewegt, während die starre Heizeinrichtung sich vorzugsweise nicht bewegt.

Steuereinrichtung

Eine bevorzugte erfindungsgemäße Vorrichtung umfasst eine Steuereinrichtung, die die Heizeinrichtung mit elektrischem Strom beaufschlagt, um das Reaktionsvolumen zu erwärmen. Die Steuerungseinrichtung ist vorzugsweise so ausgelegt, dass in mindestens einem, vorzugsweise in mindestens drei, besonders vorzugsweise in mindestens 10, besonders vorzugsweise in mindestens 20, der Durchläufe des Vervielfältigungszyklus der PCR das Verhältnis zwischen der im Denaturierungsschritt für die zum Erwärmen des Reaktionsvolumens eingesetzten elektrischen Energie und dem Fassungsvermögen des Reaktionsgefäßes weniger als 40 J/mL (Millijoule pro Milliliter), besonders vorzugsweise weniger als 20 J/mL, besonders vorzugsweise weniger als 10 J/mL, besonders vorzugsweise weniger als 3 J/mL beträgt.

Bei einer bevorzugten erfindungsgemäßen Vorrichtung ist die Steuerungseinrichtung so ausgelegt, dass mindestens ein Heizelement der Heizeinrichtung in mindestens eine Richtung eine Ausdehnung von mehr als 1,5 $\mu$m aufweist. Besonders vorzugsweise weist jedes Heizelement der Heizeinrichtung in mindestens eine, besonders vorzugsweise in zwei Richtung(en) eine Ausdehnung von mehr als 1,5 $\mu$m auf. Die Ausdehnung kann zum Beispiel eine Länge oder ein Durchmesser eines langgestreckten Heizelements sein. Ein bevorzugtes langgestrecktes Heizelement hat einen Durchmesser von mindestens 1 $\mu$m, besonders vorzugsweise von mindestens 2 $\mu$m, besonders vorzugsweise von mindestens 5 $\mu$m. Ein bevorzugtes langgestrecktes Heizelement hat eine Länge von mindestens 0,1 mm, besonders vorzugsweise von mindestens 1 mm, besonders vorzugsweise von mindestens 2 mm. Bei einem netz- oder wabenförmigen Heizelements kann die Ausdehnung zum Beispiel auch die Dicke (gemeint ist die Ausdehnung der Stege senkrecht zur Fläche des Netzes oder der Waben) oder der Durchmesser der Stege sein. Bevorzugte Stege haben eine Dicke oder einen Durchmesser von mindestens 1 $\mu$m, besonders vorzugsweise von mindestens 5 $\mu$m, besonders vorzugsweise von mindestens 10 $\mu$m.

Die bevorzugte Steuereinrichtung ist so ausgebildet, dass Sie einen Stromfluss durch die Heizeinrichtung zu Beginn des Denaturierungsschritts - oder zu Beginn jedes Zeitintervalls des Denaturierungsschritts, wenn der Denaturierungsschritt aus mehreren voneinander getrennten Zeitintervallen besteht - zulässt oder erhöht und nach Beendigung des Denaturierungsschritts - oder nach Beendigung jedes Zeitintervalls des Denaturierungsschritts - wieder unterbindet oder verringert, um einen Stromimpuls zu verursachen. Im Heizelement kann der Stromimpuls in einen Heizimpuls umgewandelt werden. Die bevorzugte Steuereinrichtung umfasst eine Stromquelle, die ihrerseits zum Beispiel ein Stromnetzteil, eine oder mehrere Batterie(n) oder Akkumulatoren oder eine Brennstoffzellen umfassen kann. Die bevorzugte Steuereinrichtung umfasst einen oder mehrere Kondensatoren. Die bevorzugte Steuereinrichtung umfasst einen Schalter, der, vorzugsweise mit wählbarer Zeitdauer, den Stromfluss aus der Stromquelle durch die Heizeinrichtung ein- und ausschalten kann. Geeignete Schalter umfassen MOSFETs, SSRs, sehr schnelle Relais und Transistoren. Die Steuereinrichtung kann zur zeitlichen Steuerung einen oder mehrere Puls- oder Frequenzgeneratoren, DACs oder Mikrocontroller aufweisen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand von Ausführungsbeispielen in den Zeichnungen schematisch dargestellt und wird im Folgenden unter Bezugnahme auf die Zeichnungen beschrieben.

Figurenbeschreibung

Die Fiuren 1A und 1B zeigen in einer schematischen Darstellung ein Heizelement gemäß einer bevorzugten Ausführungsform.

Die Figuren 2A und 2B zeigen schematisch und vereinfacht Querschnitte von bevorzugten Ausführungsformen einer Vorrichtung zum Extrahieren einer Nukleinsäure.

Figur 2C zeigt eine Probenplatte gemäß einer bevorzugten Ausfürhungsform.

Figur 2D zeigt schematisch eine Vorrichtung zum Extrahieren einer Nukleinsäure gemäß einer weiteren bevorzugten Ausführungsform.

Die Figuren 3A und 3B zeigen schematisch eine bevorzugte Ausführungsform einer Probenplatte bei der Verwendung zur Extraktion und/oder Amplifikation einer T arget-Nukleinsäure.

Figur 4 zeigt die Ergebnisse des Experiments 1 in einem Balkendiagramm.

Figur 5 zeigt die Ergebnisse des Experiments 2 in einem Balkendiagramm.

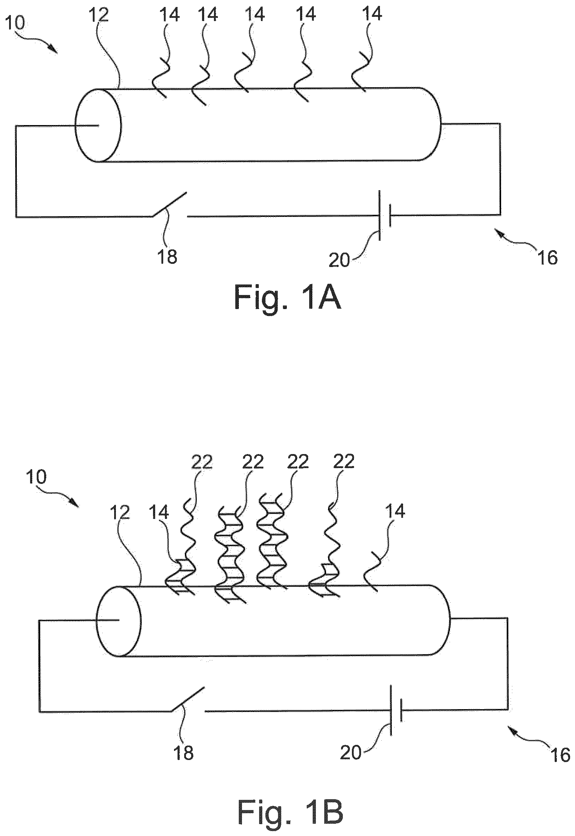

Figur 1A zeigt in einer schematischen Darstellung ein Heizelement 10 gemäß einer ersten bevorzugten Ausführungsform. Das Heizelement 10 ist als Draht bzw. Heizdraht 12 ausgebildet, der an seiner Oberfläche mit mehreren Funktionsnukleinsäuren 14 funktionalisiert ist. Dabei sei erwähnt, dass das Heizelement 10 lediglich schematisch dargestellt ist und ein tatsächlich verwendetes Heizelement 10 andere Abmessungen und/oder Formen und insbesondere ein anderes Verhältnis aus Länge zu Durchmesser aufweisen kann. Die Funktionsnukleinsäuren 14 sind als Oligonukleotide ausgebildet und weisen zumindest teilweise eine Nukleotidsequenz auf, welche zumindest teilweise komplementär zur Nukleotidsequenz zumindest eines Teils der aus einer Probenflüssigkeit zu extrahierenden Target-Nukleinsäure 22 ist. Beispielsweise können die Funktionsnukleinsäuren 14 mittels einer Thiol- und/oder Schwefel-Bindung an die Oberfläche des Heizelements 10 gebunden sein. Bevorzugt weist das Heizelement 10 eine Oberfläche auf, welche das Binden der Extraktionsnukleinsäuren 14 an das Heizelement 10 bzw. an den Draht 12 begünstigt. Beispielsweise kann das Heizelement 10 bzw. der Draht 12 aus einem Edelmetall, wie etwa aus Gold, gefertigt sein und/oder an der Oberfläche zumindest teilweise mit Gold beschichtet sein, um ein zuverlässiges Binden der Extraktionsnukleinsäuren 14 an das Heizelement 10 zu begünstigen.

Das Heizelement 10 weist eine Spannungsversorgung 16 auf, mittels welcher das Heizelement 10 mit elektrischer Spannung und/oder elektrischem Strom versorgt werden kann, um das Heizelement 10 zu beheizen und die unmittelbare Umgebung des Heizelements 10, d.h. des beheizten Drahtes 12, lokal zu erwärmen. Ferner kann bei Bedarf optional mittels des Heizelements 10 auch die gesamte Reaktionslösung, die das Heizelement 10 umgibt, global, d.h. vollständig, erwärmt werden. Beispielsweise kann durch Schließen des Schalters 18 eine von der Spannungsquelle 20 bereitgestellte elektrische Spannung an das Heizelement 10 angelegt werden, sodass ein elektrischer Strom in kontrollierbarer Weise durch das Heizelement 10 fließt und dieses resistiv beheizt. Beispielsweise kann der Strom in gepulster Form bereitgestellt werden, um zeitlich und/oder räumlich einen möglichst scharfen Temperaturgradienten in einer Reaktionslösung in der unmittelbaren Umgebung des Heizelements zu erzielen.

Zur Extraktion der Target-Nukleinsäure 22 aus einer Probenflüssigkeit kann das Heizelement 10 zumindest teilweise mit der Probenflüssigkeit bedeckt werden.

Zur Amplifikation der Target-Nukleinsäure 22 in einer Reaktionslösung kann das Heizelement 10 mit der Reaktionslösung in Kontakt gebracht werden, wie in der DE 10 2016 120 124 A1 beschreiben. Beispielsweise kann das Heizelement 10 zumindest teilweise in die Probenflüssigkeit bzw. Reaktionslösung eingetaucht werden und/oder mit der Probenflüssigkeit bzw. Reaktionslösung übergossen werden. Sofern in der Probenflüssigkeit bzw. Reaktionslösung die zu extrahierende Nukleinsäure 22 vorhanden ist und vorzugsweise frei, d.h. ungebunden, vorliegt, kann sich ein Einzelstrang der Nukleinsäure 22 an eine Funktionsnukleinsäure 14 anlegen und mit dieser hybridisieren, sofern diese noch nicht von einer anderen Nukleinsäure belegt ist. Figur 1B zeigt das Heizelement aus Figur 1A mit daran gebundenen Nukleinsäuren 22, welche aus einer Probenflüssigkeit extrahiert wurden. Die Nukleinsäuren 22 bilden mit den Extraktionsnukleinsäuren 14 zumindest teilweise Doppelstränge. Gemäß einer bevorzugten Ausführungsform kann das Heizelement 10 dazu verwendet werden, eine für die Hybridisierung der Nukleinsäure 22 mit der Extraktionsnukleinsäure 14 geeignete Temperatur in der unmittelbaren Umgebung des Heizelements 10 bereitzustellen, um etwa die Hybridisierung zu verbessern und/oder zu beschleunigen.

Wenn die Target-Nukleinsäure 22 über die Funktionsnukleinsäuren 14 an das Heizelement 10 gebunden bzw. hybridisiert sind, kann das Heizelement 10 mitsamt den Nukleinsäuren 22 wieder von der Probenflüssigkeit separiert werden, wobei die Nukleinsäuren 22 an dem Heizelement 10 verbleiben. Beispielsweise kann das Heizelement 10 aus der Probenflüssigkeit entfernt werden, wenn das Heizelement 10 darin eingetaucht wurde, und/oder die Probenflüssigkeit kann abgegossen und/oder abgesaugt werden. Auch können ein oder mehrere Waschgänge durchgeführt werden, um etwaige Rückstände von der Probenflüssigkeit, welche sich an dem Heizelement 10 abgesetzt haben können, möglichst restlos zu entfernen. Die Waschgänge sind jedoch hinsichtlich der Wasch-Reagenzien bzw. Waschlösungen und/oder hinsichtlich der Durchführung derart zu wählen, dass die Nukleinsäuren 22 auch nach den Waschgängen zumindest teileweise noch mit den Funktionsnukleinsäuren 14 an das Heizelement 10 gebunden sind.

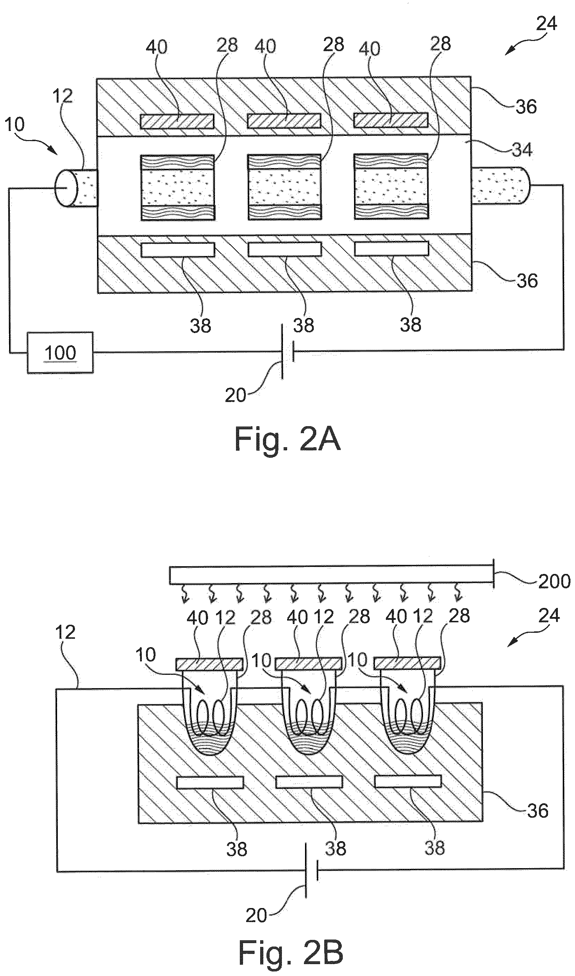

Figur 2A zeigt schematisch und vereinfacht den Querschnitt einer Ausführungsform einer Vorrichtung 24 zum Extrahieren und/oder Vervielfältigen einer Nukleinsäure 22, welche mehrere Reaktionsgefäße 28 aufweist und bei der die Heizelemente 10 von Abschnitten eines durch die mehreren Reaktionsgefäße 28 durchgehenden Drahtes 12 gebildet werden, der an eine Spannungsquelle 20 angeschlossen ist. Der Draht ist dabei mit Funktionsnukleinsäuren 14 funktionalisiert, welche jeweils sowohl als Extraktionsnukleinsäure als auch als Primer für eine Amplifikationsreaktion dienen. Der Draht 12 durchläuft mehrere voneinander getrennte Reaktionsgefäße 28 in Form von Probenflüssigkeitskammern (auch als "Wells" bezeichnet) in einer Probenplatte 34, die sich zwischen einem zweiteiligen Temperierblock 36 befindet, der als externe Heizvorrichtung dient. Der Draht 12 bildet somit in jedem der Reaktionsgefäße 28 eine eigene Heizeinrichtung bzw. ein oder mehrere eigene Heizelemente. Der Temperierblock 36 hat die Funktion, die Reaktionsvolumina in den Reaktionsgefäßen 28 auf Hybridisierungs-/Elongationstemperatur zu bringen und zu halten. Beispielsweise kann der Temperierblock 36 als ein Heizblock und/oder als ein Kühlblock ausgebildet sein. Zum In-Kontakt-Bringen und/oder Separieren der Heizelemente 10 mit der Probenflüssigkeit und/oder mit der Reaktionslösung kann die Probenflüssigkeit 26 bzw. die Reaktionslösung in die Reaktionsgefäße 28 gefüllt werden bzw. daraus abgesaugt werden.

In der hier gezeigten Ausführung befinden sich im unteren Teil des Temperierblocks 36 unter jedem Reaktionsgefäß 28 eine Anregungslichtquelle (in diesem Fall in Form einer Leuchtdiode 38 mit einem optischen Tiefpass-Filter) zum Anregen eines Farbstoffes im jeweiligen Reaktionsvolumen und im oberen Teil des Temperierblocks 36 über jeder Probenflüssigkeitskammer eine Fotodiode 40 als Lichtsensor zum Detektieren der Fluoreszenz des angeregten Farbstoffes im jeweiligen Reaktionsvolumen (mit einem optischen Hochpassfilter, der das Fluoreszenzlicht durchlässt). Diese lassen sich beispielsweise zur Detektion der amplifizierten Nukleinsäure 22 unter Verwendung von geeigneten Farbstoffen, wie etwa interkalierender Farbstoffe und/oder TaqMan-Sonden, nutzen. Die Signale der Lichtsensoren lassen sich z.B. mit einem Analog-Digitalwandler auslesen und so der zeitliche Verlauf des Fluoreszenzsignals beobachten. Insbesondere kann vorzugsweise in Echtzeit während der Durchführung der PCR das Fluoreszenzlicht als Funktion der PCR-Zyklen aufgenommen werden, um auf diese Weise eine Echtzeit-PCR ("Real-Time PCR") zu ermöglichen.

Ferner weist die Vorrichtung 24 eine Bewegungseinrichtung 100 auf, welche dazu eingerichtet ist, das Heizelement relativ zur Probenflüssigkeit bzw. relativ zur Reaktionslösung zu bewegen. Dies geschieht durch eine geeignete ung des Heizelements 10 vorzugsweise in Verbindung mit einem optionalen externen Magnetfeld (nicht gezeigt) und/oder mit dem durch andere bestromte Heizelemente erzeugten Magnetfeld. Durch die geeignete Bestromung erzeugt der Stromfluss durch das Heizelement ein magnetfeld, welches mit dem externen Magnetfeld wechselwirkt und zu einer Bewegung des Heizelements relativ zur Probenflüssigkeit bzw. relativ zur Reaktionslösung führt.

Figur 2B zeigt schematisch und vereinfacht den Querschnitt einer Vorrichtung 24 zum Extrahieren einer Nukleinsäure 22 gemäß einer weiteren bevorzugten Ausführungsform, die sich von dem Ausführungsbeispiel der Figur 2A dadurch unterscheidet, dass die Heizelemente 10 als Spulen aus Abschnitten eines an eine Spannungsquelle 20 angeschlossen Drahts 12 ausgeführt sind. Die Heizelemente 10 in Form von zu Spulen aufgewickelten Drahts 12 stehen mit dem Reaktionsvolumen in dem jeweiligen Reaktionsgefäß 28 in Kontakt. Anders als in der Figur dargestellt, sind sie vorzugsweise vollständig von der Reaktionslösung bzw. der Probenflüssigkeit umgeben. Die Reaktionsgefäße 28 sind in diesem Ausführungsbeispiel als mehrere voneinander getrennte Probenflüssigkeitskammern in Form von Reaktionstubes ausgebildet, die sich in einem Temperierblock 36 befinden, um die Reaktionsvolumina auf Hybridisierung/-Elongationstemperatur zu bringen und zu halten. In der hier gezeigten Ausführung befindet sich im unteren Teil des Temperierblocks 36 unter jeder Probenflüssigkeitskammer eine Leuchtdiode 38 als Anregungslichtquelle zum Anregen eines Farbstoffes im Reaktionsvolumen und über jeder Probenflüssigkeitskammer eine Fotodiode 40 als Lichtsensor zum Detektieren der Fluoreszenz des angeregten Farbstoffes im Reaktionsvolumen.

Ferner weist die Vorrichtung 24 eine Bewegungseinrichtung 200 auf. Diese ist dazu eingerichtet, die in den Reaktionsgefäßen 28 befindliche Probenflüssigkeit oder Reaktionslösung mit Schallwellen und/oder Ultraschallwellen zu beaufschlagen und auf diese Weise relativ zu den Heizelementen 10 zu bewegen.

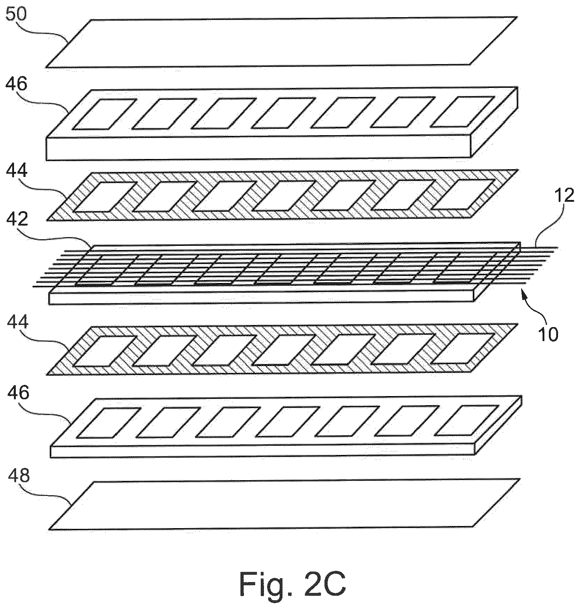

Figur 2C zeigt schematisch Komponenten, aus denen eine Probenplatte einer Vorrichtung 24 zum Extrahieren und/oder Vervielfältigen einer Nukleinsäure 22 gemäß einer bevorzugten Ausführungsform mit Draht-Heizelementen 10 geschaffen werden kann. Als Heizelemente 10 dienen hier Abschnitte eines vergoldeten Mantel-Drahts 12 von 25 $\mu$m Durchmesser (24,8 $\mu$m Wolfram-Kern mit ca. 100 nm Gold-Mantel, LUMA-METALL AB, Kalmar, Schweden). Dieser wird um eine Acrylglasplatte 42 mit einer Dicke von 0,5 mm gewickelt (mittlere, hell dargestellte Platte). In der Acrylglasplatte 42 sind sieben Öffnungen (6 mm x 6 mm) vorhanden, durch welche die Reaktionsgefäße 28 bzw. Probenflüssigkeitskammern (Wells) gebildet werden. Durch die Wicklung des Drahtes 12 entstehen in jedem Reaktionsgefäß 28 (diese sind nur bei zusammengesetzter Vorrichtung erkennbar) zwei parallele Schichten aus je 25 parallelen Heizelementen 10 (auch eine andere Zahl an Heizelementen zwischen typischerweise 10 und 75 Heizelementen je Lage können vorteilhaft sein). Die beiden Lagen von Heizelementen 10 haben durch die Platte einen Abstand von 0,7 mm voneinander, die Heizelemente 10 innerhalb einer Schicht haben einen Abstand von ca. 0,24 mm voneinander. Von beiden Seiten wird beispielsweise mithilfe von beidseitig klebenden Folien 44 (dunkel dargestellt, 100-250 $\mu$m starkes VHB Klebeband von 3M) mit entsprechenden Aussparungen für die Probenflüssigkeitskammern auf die umwickelte Platte 42 je eine weitere Acrylglasplatte 46 (Dicke der unteren Platte 0,5 mm und Dicke der oberen Platte 3 mm) mit gleichen Öffnungen aufgeklebt und gemäß den Herstellerangaben des Klebebands 44 verpresst. Von unten sind die Wells bzw. Reaktionsgefäße z.B. mit einer dünnen Folie 48 (hell dargestellt, Adhesive PCR Foil Seal, 4titute) verschlossen, die auf die untere Acrylglasplatte 46 aufgeklebt ist. Auf diese Weise entsteht eine Probenplatte mit sieben Wells, die von parallelen Drähten 12 als Heizelemente 10 durchzogen werden. Die Drähte 12 werden an den beiden außenliegenden Enden der Probenplatte miteinander verbunden (das heißt alle Drähte/Heizelement sind parallel geschaltet) und elektrisch kontaktiert. Dadurch wird ermöglicht, dass Stromimpulse seriell durch die Heizelemente allet Wells bzw. Reaktionsgefäße 28 geschickt werden können. Die Öffnungen der Probenplatte (hier oben) können anschließend mit einer dünnen Folie 50 (hell dargestellt) verschlossen werden. Die Probenplatten haben gemäß dieser bevorzugten Ausführungsform eine Breite von 20 mm und eine Länge von 90 mm (so dass die Spannung der Heizpulse im Wesentlichen über eine Länge von ca. 96 mm abfällt, wenn man die 3 mm Überstand der Drähte 12 an den Enden der Probenplatte berücksichtigt, die für eine Kontaktierung erforderlich sind). Typischerweise ergibt sich ein elektrischer Widerstand von insgesamt ca. 250 Milliohm über die Länge der Probenplatte (mit 50 parallel geschalteten Drähten).

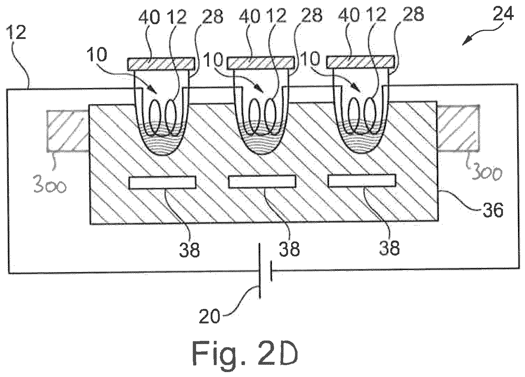

Figur 2D zeigt schematisch eine Vorrichtung 24 zum Extrahieren einer Nukleinsäure gemäß einer weiteren bevorzugten Ausführungsform, welche in vielen Aspekten der Vorrichtung 24 aus Figur 2B entspricht. Jedoch liegt ein Unterschied darin, dass die Vorrichtung 24 gemäß Figur 2D eine Bewegungseinrichtung 300 aufweist, welche als Vibrationsvorrichtung ausgebildet ist. Die Bewegungseinrichtung 300 ist seitlich an dem Temperierblock 36 ausgebildet und umfasst gemäß der gezeigten Ausführungsform zwei Vibrationselemente, wie etwa Piezo-Aktuatoren, die in direktem mechanischen Kontakt mit dem Temperierblock 36 angeordnet sind. Die Bewegungseinrichtung 300 ist dazu eingerichtet, den Temperierblock 36 und über diesen die Reaktionsgefäße 28 derart zu Schwingungen und/oder Virbationen anzuregen, dass die in den Reaktionsgefäßen 28 angordnete Probenflüssigkeit 500 bzw. Reaktionslösung 400 und/oder die in den Reaktionsgefäßen 28 angeordneten Heizelemente 10 zum Schwingen und/oder Vibrieren angeregt werden. Die Bewegungseinrichtung 300 kann vorzugsweise noch weitere Elemente aufweisen, welche beispielsweise dazu dienen können, die Vibrationselemente mit Energie zu versorgen und/oder zu steuern und/oder zu regeln.

Gemäß anderen bevorzugten Ausführungsformen können die Bewegungseinrichtung bzw. die Vibrationseinrichtung bzw. die Vibrationselemente beispielsweise unterhalb des Temperierblocks 36 und/oder oben auf dem Temperierblock 36 und/oder auf den Reaktionsgefäßen 28 ausgebildet sein. Gemäß anderen Ausführungsform kann die Bewegungseinrichtung 300 lediglich ein Vibrationselement aufweisen oder für jedes Reaktionsgefäß 28 eine eigenes Vibrationselement.

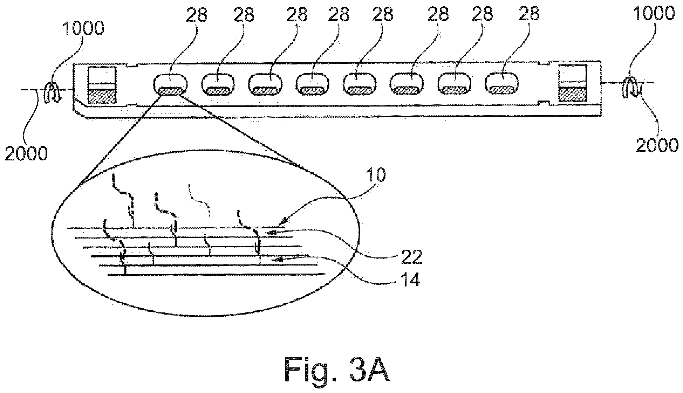

Fig. 3A zeigt in einer schematischen Darstellung eine bevorzugte Ausführungsform der Erfindung. Gemäß dieser Ausführungsform wird eine Probenplatte, wie in Figur 2C dargestellt, verwendet, welche mehrere Reaktionsgefäße 28 umfasst, verwendet.

Die Probenplatte ist dabei derart ausgestaltet, wie beispielsweise in Figur 2C dargestellt und erläutert. Gemäß dieser Ausführungsform ist die Probenplatte mittels einer Bewegungseinrichtung (nicht gezeigt) insbesondere während der Inkubationszeit (gemeinsam mit dem Heizblock) um einen Winkel 1000 schwenkbar, der vorzugsweise +/-10°, besonders vorzugsweise +/-20°, besonders vorzugsweise +/-30°, besonders vorzugsweise +/-40°, besonders vorzugsweise +/-50°, besonders vorzugsweise +/-60°, besonders vorzugsweise +/-70° besonders vorzugsweise +/-80°, besonders vorzugsweise +/-90°, und besonders vorzugsweise mehr als +/- 90° mit einer Frequenz von vorzugsweise 1 mal pro Minute, vorzugsweise 3 mal pro Minute besonders vorzugsweise 10 mal pro Minute, vorzugsweise 20 mal pro Minute, vorzugsweise 30 mal pro Minute, vorzugsweise 60 mal pro Minute um vorzugsweise eine Symmetrieachse der Probenplatte, besonders vorzugsweise zwei Symmetrieachse der Probenplatte und besonders vorzugsweise drei Symmetrieachse der Probenplatte hin und her gekippt wird, so dass die Reaktionslösung in den Reaktionskammern hin und her schwappt und auf diese Weise eine Relativbewegung zwischen Heizelementen (vorzugsweise Drähte) und der Reaktionslösung/Probenflüssigkeit hervorgerufen wird. Es kann hierdurch erreicht werden, dass bei geringen Konzentrationen (z.B. weniger als vorzugsweise 1000 oder vorzugsweise 100 oder vorzugsweise 10 Kopien oder vorzugsweise 5 Kopien) der zu vervielfältigenden Nukleinsäure, bei einem höheren Prozentsatz positive Real-Time-PCR Kurven in der nachfolgenden Amplifikationsreaktion gemäß DE 10 2016 120 124.3 erreicht werden. Dies wird erfindungsgemäß darauf zurückgeführt, dass mehr Bindungs-/Einfang-/Hybridisierungsereignisse von Nukleinsäuren an die funktionalisierten Heizelemente durch die von außen induzierte Relativbewegung zwischen Heizelementen und Reaktions- bzw. Probenflüssigkeit hervorgerufen wird.

Im unteren Bereich der Figur 3A befindet sich eine vergrößerte, schematische Darstellung der Heizelemente 10, welche die Reaktionsgefäße 28 durchziehen und mit Funktionsnukleinsäuren 14 und Target-Nukleinsäuren 22 verbunden sind.

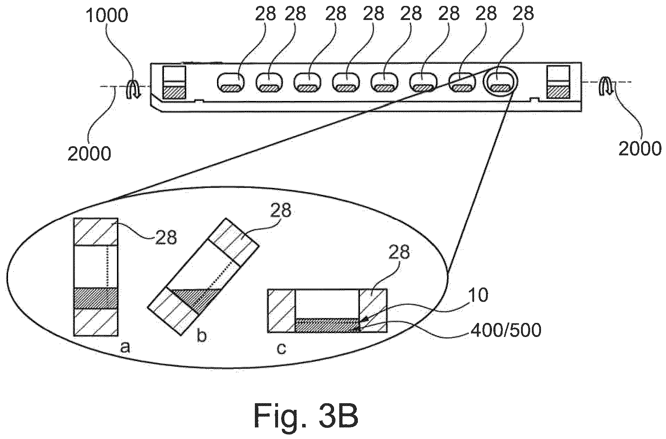

In Figur 3B wird die durch die Bewegungsvorrichtung hervorgerufene Kippbewegung gemäß der in Figur 3A gezeigten bevorzugten Ausführungsform während der Hybridisierung gemäß einem Ausführungsbeispiel genauer erläutert, ohne jedoch darauf beschränkt zu sein. Die Probenplatte, deren Reaktionskammern 28 mit der Reaktionslösung 400 oder mit der Probenflüssigkeit 500 befüllt sind, wird in die Vorrichtung 24 eingelegt und durch einen Heizblock von unten mit 56 °C und von oben mit 63 °C beheizt. Die gesamte Vorrichtung, die die Probenplatte enthält, wird nun für fünf Minuten folgendermaßen bewegt: Mit einer Frequenz von 20 mal pro Minute wird die gesamte Vorrichtung um einen Winkel 1000 von +90 ° entlang der Längsachse 2000 der Probenplatte gekippt und wieder in die Ausgangsposition zurückgebracht. Von der Ausgangsposition aus wird nun um einen Winkel 1000 von -90 ° entlang der Längsachse 2000 der Probenplatte gekippt und anschließend die Vorrichtung mit der Probenplatte wieder in die Ausgangsposition zurückgebracht. Nachdem die gesamte Vorrichtung fünf Minuten derartig bewegt wurde, wird die Probenplatte aus der Vorrichtung 24 entfernt und die Probenplatte bei Raumtemperatur weitere zwei Minuten mit einer Frequenz von 20 mal pro Minute wie oben beschrieben bewegt.

In der unteren Skizze ist die Bewegung der Reaktionslösung während einer +90 ° Kippbewegung (ohne Rückkehr in die Ausgangsposition) dargestellt.

Im Unteren Bereich der Figur 3B ist ferner ein Reaktionsgefäß bzw. -kammer 28 inklusive der Heizelemente 10 und der Reaktionslösung in drei verschiedenen Kipplagen a, b und c gezeigt. Durch die Kippbewegung der Probenplatte schwappt in der Reaktionskammer 28 die Reaktionslösung 400 bzw. Probenflüssigkeit 500 hin und her, wodurch eine Relativbewegung zwischen Heizelementen 10 und Reaktionslösung 400 bzw. Probenflüssigkeit 500 hervorgerufen wird.

Im Folgenden werden beispielhaft mit bevorzugten Ausführungsformen der Erfindung durchgeführte Experimente erläutert, ohne dass die Erfindung jedoch auf diese Beispiele beschränkt ist.

Insbesondere zeigen die im Folgenden Experimente, dass eine Relativbewegung der Flüssigkeit, d.h. der Probenflüssigkeit bzw. der Reaktionslösung, und der Heizelemente eine Erhöhung der Bindungseffizienz der Target-Nukleinsäure an die Funktionsnukleinsäuren auf den Heizelementen verursacht.

Experiment 1 - Relativbewegung mittels Ultraschall

In diesem Experiment wird zur Erzeugung der Relativbewegung zwischen der Flüssigkeit, d.h. der Probenflüssigkeit und/oder der Reaktionslösung, und den Heizelementen die Flüssigkeit mit Ultraschall beaufschlagt und die an die Funktionsnukleinsäuren gebundenen Target-Nukleinsäuren ermittelt. Dies wird sodann mit einer Vergleichsmessung verglichen, in welcher keine Ultraschallbeaufschlagung erfolgte, aber ansonsten eine identische Messung durchgeführt wurde.

Die Reaktionsgefäße werden als Reaktionsplatten mit mehreren Reaktionskammern bereitgestellt und sind, wie in der Patentanmeldung DE 10 2016 120 124 A1 beschrieben, mit Drähten als lokale Heizelemente bestückt, wobei in dem vorliegenden Experiment 20 goldbeschichtete Drähte mit einer Länge von 6 mm pro Reaktionskammer bzw. Reaktionsgefäß vorhanden sind. Die hier benutzten Heizelemente sind als Drähte ausgebildet, haben einen KernDurchmesser von 14,6 μm aus Wolfram und sind mit 200nm Gold beschichtet (LUMA METALL, Schweden) und haben zueinander einen Abstand von 0,076 mm. Die Heizelemente verlaufen ca. 0,7mm über dem Boden des Reaktionsgefäßes, das eine Grundfläche von 6 mm x 6mm hat, so dass es bei einer Befüllung mit 60μl Probenflüssigkeit bis zu einer Höhe von ca. 1,7 mm (über dem Boden) befüllt ist. Die Heizelemente verlaufen durchgehend durch mehrere nebeneinander liegende Probenkammern bzw. Reaktionsgefäße und haben im gesamten Probenträger insgesamt jeweils eine Länge von 10cm (pro Probenkammer bzw. Reaktionsgefäß jedoch jeweils nur 6mm).

Die Funktionalisierung der Funktionsnukleinsäuren auf die goldbeschichteten Drähte (Heizelemente) wurde wie in der DE 10 2016 120 124 A1 beschrieben durchgeführt. Hier kurz erwähnt: Eine Funktionalisierungslösung enthält 100nM Funktionsnukleinsäure und 100 mM MgCl2 in Phosphat-Puffer. 50μl hiervon wurde pro Reaktionsgefäß eingesetzt und über Nacht bei 4°C inkubiert. Diese Funktionalisierungslösung würde am folgenden Tag aus den Reaktionsgefäßen entfernt und anschließend wurden die Reaktionsgefäße dreimal mit ausreichend Wasser gewaschen. Danach wurde die freie Oberfläche der Heizelemente mit 3% Plasmalösung 10 min bei Raumtemperatur abgesättigt, um unspezifische Bindungen der Target-Nukleinsäure auf die Gold-Oberfläche der Heizelemente zu vermeiden. Den Überschuss an Plasmalösung wurde dreimal mit ausreichend Wasser ausgewaschen.

Für die Hybridisierung der Target-Nukleinsäure an die Funktionsnukleinsäure wurde die Target-Nukleinsäure (extrahierte genomische DNA aus MRSA (Methicillin-resistenter Staphylococcus aureus) mit Hybridisierungspuffer (kommerziell erhältlicher AL-Puffer von QUIAGEN, Endkonzentration 25%) gemischt. Jeweils 60μl des Hybridisierungspuffers mit der Target-Nukleinsäure wurde in die Reaktionsgefäße pipettiert und mit einer drucksensitiven Folie dicht versiegelt (5000 Kopien genomischer DNA pro Reaktionsgefäß), so dass sich alle Heizelemente innerhalb der Reaktionsgefäße (vollständig) in Kontakt der Lösung befinden.

Die Reaktionsgefäße wurden dann in einem Ultraschall-Bad (Typ UMD030, Hersteller EUMAX, 260W Gesamtleistung) bis zum Boden versenkt und in Kontakt mit dem Boden des Ultraschall-Bades 10 min bei 25°C beschallt, wodurch eine Relativbewegung zwischen der Flüssigkeit in den Reaktionsgefäßen und den Heizelementen hervorgerufen wurde. Anschließend wurden die Reaktionsgefäße aus dem Ultraschallbad genommen und zügig nach Entfernung der Folie die Lösung aus Hybridisierungspuffer und nicht gebundene Ziel-Target heraus pipettiert bzw. entnommen. Die Reaktionsgefäße wurden dann dreimal mit Waschpuffer (basiert auf 9mM MgCl2 und 10mM Tris pH: 8) bei Raumtemperatur gewaschen.

Anschließend wurden alle Heizelemente aus den Reaktionsgefäßen herausgeschnitten und mit einer Pinzette in ein PCR-Tube überführt. Diese Heizelemente wurden dann mit ausreichend PCR-Mastermix abgedeckt. Der PCR-Mastermix enthielt spezifische Primer und Probes für die Target-Nukleinsäure, eine DNA-Polymerase, dNTPs und die üblichen Komponenten eines klassischen real-time PCR Mixes. Die anschließende qPCR wurde in einem LightCylcer Instrument (ROCHE) durchgeführt, um die Menge an Target-Nukleinsäure zu bestimmen, die zuvor an die Funktionsnukleinsäure und die Heizelemente gebunden hat.

Als Referenz bzw. Vergleichsmessung wurden eine weitere Reaktionsplatte bzw. weitere Reaktionsgefäße herangezogen, die ansonsten gleich wie in der vorherigen Messung behandelt wurden, jedoch statt 10 Minuten im Ultraschallbad lediglich 10 Minuten ohne Bewegung und somit ohne herbeigeführte Relativbewegung zwischen der Flüssigkeit und den Heizelementen bei Raumtemperatur inkubiert wurde. In diesen Reaktionsgefäßen zeigte sich, dass nur ca. 2% der angebotenen 5000 Kopien genomischer DNA pro Reaktionsgefäß (Target-Nukleinsäure) an die Funktionsnukleinsäuren auf den Heizelementen gebunden hat, während in den Reaktionsgefäßen, die im Ultraschall-Bad zur Herbeiführung der Relativbewegung behandelt wurden, ca. 19% der angebotenen 5000 Kopien genomischer DNA pro Reaktionsgefäß (Target-Nukleinsäure) an die Funktionsnukleinsäure auf den Heizelementen gebunden hat.

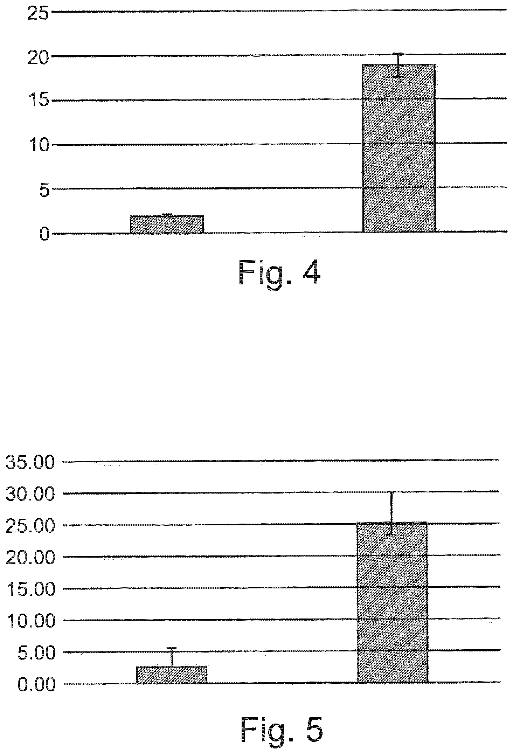

Die Ergebnisse sind grafisch in dem Balkendiagramm Figur 4 dargestellt. Auf der y-Achse ist der Anteil der eingesetzten Taget-Nukleinsäure, der an die Funktionsnukleinsäuren gebunden hat, in %-Angaben dargestellt. Der linke Balken gibt die Ergebnisse der Vergleichsmessung ohne Ultraschall-Beaufschlagung wieder und lässt einen Anteil von ca. 2% an gebundenen Target-Nukleinsäuren erkennen. Der rechte Balken zeigt das Ergebnis der Messung, bei welcher mittels Ultraschallbeaufschlagung eine Relativbewegung herbeigeführt wurde. Hier liegt der Anteil mit ca. 19% deutlich höher, als in der Vergleichsmessung.

Es konnte demnach gezeigt werden, dass die Extraktionseffizienz um ca. einen Faktor 10 erhöht werden konnte. Entsprechend ist zu erwarten, dass auch die Sensitivität einer solchen Vorrichtung und/oder Methode zur Extraktion und/oder zur Vervielfältigung und/oder zum Nachweis einer Target-Nukleinsäure mindestens um einen Faktor 10 verbessert werden kann, im Vergleich zu herkömmlichen Ausführungsformen ohne Relativbewegung.

Experiment 2 - Relativbewegung mittels schwingender Heizelemente

In diesem Experiment wurden ähnliche Vorbereitungen getroffen, wie bereits oben mit Bezug auf das Experiment 1 erläutert. Abweichend vom Experiment 1 wurde jedoch die Anzahl der Heizelemente (Drähte) pro Reaktionskammer wurde von 20 auf 75 erhöht, wobei jedoch der Abstand unverändert zum Experiment 1 war. Die Funktionalisierungslösung enthält hier 500nM Funktionsnukleinsäure und 500 mM MgCl2 in Phosphat-Puffer. 50$\mu$l hiervon wurde pro Reaktionsgefäß eingesetzt und lediglich 10 Minuten bei Raumtemperatur inkubiert. Zudem wurden hier pro Reaktionsgefäß statt 5000 Kopien nur 500 Kopien genomischer DNA eingesetzt.

An den Enden der Drähte bzw. Heizelemente, die durch die den Probenträger und somit durch die Reaktionsgefäße verlaufen, wurden für drei Minuten eine Wechselspannung angelegt (Rechteckform, 2 V Peak-to-Peak, Offset 1V, DutyCylce 50% Frequenz 4,5 kHz, wobei die Spannung über der gesamten Länge von 10cm der Heizelemente bzw. Drähte anlag). Die Reaktionsplatte bzw. die Reaktionsgefäße selbst wurden bei Raumtemperatur gehalten. Über und unter den jeweiligen Reaktionsgefäßen befanden sich Neodym-Eisen-Bor (NdFeB, N42) Permenentmagneten (Durchmesser ca. 5mm; Höhe, ca. 8mm). Der Abstand zwischen dem Magneten über der Reaktionskammer und dem entsprechenden Magneten darunter betrug ca. 5mm. Während die pulsierende Gleichspannung angelegt war, war ein akustisches Summen vernehmbar, das als Hinweis auf eine Schwingung der Drähte bzw. der Heizelemente in den Reaktionsgefäßen und somit auf die Bewegung der Heizelemente angesehen wird. Das Summen wurde insebesondere durch die externen Permanentmagneten verstärkt, da es leiser wurde, wenn die Magneten testweise entfernt wurden. Während der Strom durch die Heizelemente fließt, entstehen Magnetfelder um die Heizelemente bzw. Drähte, die zu einer Abstoßung der Heizelemente bzw. Drähte untereinander führen (insbesondere für die Drähte, die am Rand liegen, und daher keine oder eine geringere Anzahl von benachbarten Drähten haben), so wie zu einer Wechselwirkung mit dem externen Magnetfeld gemäß der Lorentzkraft, die auf stromdurchflossene Leiter in einem Magnetfeld wirkt. Durch verschiedene Orientierungen des Magnetfelds können vorzugsweise Bewegungen der stromdurchflossenen Drähte entlang unterschiedlicher Richtungen erzeugt werden. Anschließend wurde nach Entfernung der Folie die Lösung aus Hybridisierungspuffer und nicht gebundener Target-Nukleinsäure aus den Reaktionskammern pipettiert. Die Reaktionskammern wurden dann dreimal mit Waschpuffer (basiert auf 9mM MgCl2 und 10mM Tris pH: 8) bei Raumtemperatur gewaschen.

Anschließend wurden alle Heizelemente aus jedem Reaktionsgefäß herausgeschnitten und mit einer Pinzette in ein PCR-Tube überführt, um mit einem konventionen qPCR Thermocycler (Roche Lightcycler) eine Quantifizierung der DNA auf den Heizelementen durchzuführen. Diese Heizelemente wurden dann mit ausreichend PCR-Mastermix in den PCR-Tubes abgedeckt. Der PCR-Mastermix enthaltet die Target-Spezifische Primer und Probenukleinsäuren, eine DNA-Polymerase, dNTPs und die üblichen Komponenten eines klassischen real-time PCR Mixes. Die anschließende qPCR wurde in einem LightCylcer Instrument (ROCHE) durchgeführt, um die Menge an Target-Nukleinsäure zu bestimmen, die zuvor an die Funktionsnukleinsäure gebunden hat.

Als Referenz bzw. Vergleichsmessung wurden eine weitere Reaktionsplatte bzw. weitere Reaktionsgefäße herangezogen, die gleich wie in der vorherigen Messung behandelt wurden, jedoch statt drei Minuten mit Spannungsbeaufschlagung zur Schwingungsanregung lediglich drei Minuten ohne Bewegung bzw. Schwinung bei Raumtemperatur inkubiert wurde. In dieser Reaktionslplatte zeigt sich, dass nur ca. 3% der angebotenen 500 Kopien genomischer DNA pro Reaktionskammer (Target-Nukleinsäure) an die Funktionsnukleinsäure auf den Drähten gebunden hat, während auf der Reaktionsplatte mit den Reaktionsgefäßen, deren Heizelemente zur Schwingungsanregung mit der pulsierende Gleichspannungbeaufschlagt wurden, ca. 25% der angebotenen 500 Kopien genomischer DNA (Target-Nukleinsäure) pro Reaktionsgefäß an die Funktionsnukleinsäure auf den Heizelementen gebunden hat.

Die Ergebnisse sind grafisch in dem Balkendiagramm Figur 5 dargestellt. Auf der y-Achse ist der Anteil der eingesetzten Taget-Nukleinsäure, der an die Funktionsnukleinsäuren gebunden hat, in %-Angaben dargestellt. Der linke Balken gibt die Ergebnisse der Vergleichsmessung ohne Schwingungsanregung der Heizelemente mittels elektrischer Spannung wieder und lässt einen Anteil von ca. 3% an gebundenen Target-Nukleinsäuren erkennen. Der rechte Balken zeigt das Ergebnis der Messung, bei welcher mittels der pulsierenden Gleichspannung die Heizelementen zum Schwingen angeregt wurden und dadurch eine Relativbewegung zwischen den Heizelementen und der Flüssigkeit herbeigeführt wurde. Hier liegt der Anteil mit ca. 25% deutlich höher als in der Vergleichsmessung.

Es konnte demnach gezeigt werden, dass die Extraktionseffizienz um mehr als einen Faktor 8 erhöht werden konnte. Entsprechend ist zu erwarten, dass auch die Sensitivität einer solchen Vorrichtung und/oder Methode zur Extraktion und/oder zur Vervielfältigung und/oder zum Nachweis einer Target-Nukleinsäure mindestens um einen Faktor 8 verbessert werden kann, im Vergleich zu herkömmlichen Ausführungsformen ohne Relativbewegung.

In beiden Experimenten wurde eine Funktionsnukleinsäure mit einer Sequenz verwendet, die am 3'-Ende spezifisch für MRSA ist (die Funktionen der anderen Elemente sind entsprechend der Druckschrift DE 10 2016 120 124 A1 zu entnehmen:
5'Thiol - AAAAAAAAAAAAAAAAAAAAAAAAAAAAAAAAAAAA [Seq. ID 1] /iSp9/ AAATGATTATGGCTCAGGTACTGC [Seq. ID 2]

Als Primer für die qPCR im LightCycler wurde ein Primer/Probe-set verwendet, das ebenfalls spezifisch für MRSA ist:

Forward-Primer: AAATGATTATGGCTCAGGTACTGC [Seq. ID 3]
Reverse-Primer: TGAAGATGTGCTTACAAGTGCTA [Seq. ID 4]
Taqman-Probe: 5'FAM-TCCACCCTCAAACAGGTGAATTAT [Seq. ID 5]-3'BHQ1
iSp9 enstpricht der abasischen Modifikation Spacer9.
5'FAM entspricht Fluorescein Amidites.
3'BHQ1 entspricht dem Black Hole Quencher-1

Bezugszeichen

10 Heizelement
12 Heizdraht
14 Funktionsnukleinsäure
16 Spannungsversorgung
18 Schalter
20 Spannungsquelle
22 Target-Nukleinsäure
24 Vorrichtung zum Extrahieren/Amplifizieren einer Nukleinsäure
28 Reaktionsgefäß
34 Probenplatte
36 Temperierblock
38 Leuchtdiode
40 Fotodiode
42 Acrylglasplatte
44 Klebeband
46 Acrylglasplatte
48 dünne Folie
50 dünne Folie

100 Bewegungseinrichtung
200 Bewegungseinrichtung
300 Bewegungseinrichtung

400 Reaktionslösung
500 Probenflüssigkeit

1000 (Schwenk-) Winkel
2000 Längsachse der Probenplatte

**Patentansprüche**

1. Verfahren zum Extrahieren einer Target-Nukleinsäure (22) aus einer Probenflüssigkeit (500), umfassend die Schritte:

- Bereitstellen einer Heizeinrichtung mit einem Heizelement (10) in Kontakt mit der Probenflüssigkeit (500), wobei das Heizelement (10) mit zumindest einer Funktionsnukleinsäure (14) konjugiert ist und wobei die zumindest eine Funktionsnukleinsäure (14) dazu ausgelegt ist, mit der Target-Nukleinsäure (22) zu hybridisieren und die Target-Nukleinsäure (22) an das Heizelement (10) zu binden;
- Erzeugen einer Relativbewegung zwischen dem Heizelement (10) und der Probenflüssigkeit (500);
- Extrahieren der Target-Nukleinsäure (22) aus der Probenflüssigkeit (500) durch ein Separieren des Heizelements (10) von der Probenflüssigkeit (500).

2. Verfahren zum Vervielfältigen einer Target-Nukleinsäure (22), umfassend die Schritte:

- Extrahieren der Target-Nukleinsäure (22) aus einer Probenflüssigkeit (500) durch Bereitstellen einer Heizeinrichtung mit einem Heizelement (10) in Kontakt mit der Probenflüssigkeit (500), wobei das Heizelement (10) mit zumindest einer Funktionsnukleinsäure (14) konjugiert ist und wobei die zumindest eine Funktionsnukleinsäure (14) dazu ausgelegt ist, mit der Target-Nukleinsäure (22) zu hybridisieren und die Target-Nukleinsäure (22) an das Heizelement (10) zu binden;
- Vervielfältigen der aus der Probenflüssigkeit (500) extrahierten Target-Nukleinsäure (22) mittels einer Polymerase-Kettenreaktion in einer Reaktionslösung (400) unter Verwendung der Heizeinrichtung;

wobei das Extrahieren der Target-Nukleinsäure (22) ferner ein Erzeugen einer Relativbewegung zwischen dem Heizelement (10) und der Probenflüssigkeit (500) und ein Separieren des Heizelements (10) von der Probenflüssigkeit (500) umfasst.

3. Verfahren gemäß Anspruch 2, wobei das Vervielfältigen der Target-Nukleinsäure (22) ferner ein Erzeugen einer Relativbewegung zwischen dem Heizelement (10) und der Reaktionslösung (400) umfasst.

4. Verfahren nach einem der vorhergehenden Ansprüche, wobei das Erzeugen der Relativbewegung zwischen dem Heizelement (10) und der Probenflüssigkeit (500) dann erfolgt, wenn das Heizelement (10) mit der Probenflüssigkeit (500) in Kontakt ist, und/oder wobei das Erzeugen der Relativbewegung zwischen dem Heizelement (10) und der Reaktionslösung (400) zumindest teilweise während eines Denaturierungsschrittes der Polymerase-Kettenreaktion erfolgt.

5. Verfahren gemäß einem der vorhergehenden Ansprüche, wobei zumindest während des Erzeugens der Relativbewegung zwischen dem Heizelement (10) und der Probenflüssigkeit (500) und/oder während des Erzeugens der Relativbewegung zwischen dem Heizelement (10) und der Reaktionslösung (400) zumindest zeitweise eine für die Hybridisierung der Target-Nukleinsäure (22) mit der Funktionsnukleinsäure (14) geeignete Temperatur zumindest am Ort der Funktionsnukleinsäure (14) bereitgestellt wird, und wobei die Temperatur zumindest 20° C, bevorzugt zumindest 30° C mehr bevorzugt zumindest 40° C, viel mehr bevorzugt zumindest 50° C und am meisten bevorzugt zumindest 60° C beträgt und/oder die Temperatur nicht mehr als 90° C, bevorzugt nicht mehr als 80° C, am meisten bevorzugt nicht mehr als 70° C beträgt, und wobei ein Bereitstellen von Wärme für das Bereitstellen der Temperatur optional zumindest teilweise mittels des Heizelements (10) erfolgt.

6. Verfahren gemäß einem der vorhergehenden Ansprüche, wobei die Funktionsnukleinsäure (14) als eine Extraktionsnukleinsäure ausgebildet ist und/oder eine Fänger-Nukleotidsequenz aufweist, welche zumindest teilweise komplementär zur Nukleotidsequenz der Target-Nukleinsäure (22) ist und dazu geeignet ist, an die Target-Nukleinsäure (22) zu binden.

7. Verfahren gemäß einem der vorhergehenden Ansprüche, wobei die Funktionsnukleinsäure (14) eine Nukleotidsequenz aufweist, welche als Primersequenz für die Vervielfältigung der Target-Nukleinsäure (22) verwendbar ist.

8. Verfahren gemäß einem der vorhergehenden Ansprüche, wobei die Relativbewegung zwischen dem Heizelement (10) und der Probeflüssigkeit (500) und/oder der Reaktionslösung (400) zumindest teilweise durch ein Bewegen des Heizelements (10) erfolgt.

9. Verfahren gemäß Anspruch 8, wobei das Bewegen des Heizelements (10) ein Schwingen und/oder ein Vibrieren des Heizelements (10) umfasst und wobei das Schwingen und/oder Vibrieren des Heizelements (10) optional mittels eines Magnetfeldes hervorgerufen wird, wobei das Magnetfeld vorzugsweise durch einen elektrischen Stromfluss im Heizelement (10) und/oder durch ein externes Magnetfeld erzeugt wird.

10. Verfahren gemäß Anspruch 9, wobei das Schwingen und/oder Vibrieren des Heizelements (10) zumindest teilweise durch eine Krafteinwirkung durch einen mechanischen Aktor und/oder durch Schallwellen und/oder Ultraschallwellen hervorgerufen wird.

11. Verfahren gemäß einem der vorhergehenden Ansprüche, wobei das eine oder die mehreren Heizelemente (10) als elektrische und insbesondere als resistive Heizelemente (10) ausgebildet sind und bevorzugt als Heizdraht (12) bzw. Heizdrähte (12) ausgebildet sind, und wobei die Heizeinrichtung optional mehrere Heizelemente (10) aufweist.

12. Verfahren gemäß einem der vorhergehenden Ansprüche, wobei das Heizelement (10) mit einer Mehrzahl von Funktionsnukleinsäuren (14) konjugiert ist, wobei die Funktionsnukleinsäuren (14) des Heizelements (10) gleichartig oder zumindest teilweise verschiedenartig ausgebildet sind.

13. Verfahren gemäß Anspruch 11, wobei jedes der Heizelemente (10) jeweils mit einer Mehrzahl von gleichartigen Funktionsnukleinsäuren (14) konjugiert ist und die verschiedenen Heizelemente (10) vorzugsweise mit Funktionsnukleinsäuren (14) konjugiert sind, die von den Funktionsnukleinsäuren (14) der anderen Heizelemente (10) zumindest teilweise verschieden sind.

14. Vorrichtung (24) zum Extrahieren und/oder Vervielfältigen einer Target-Nukleinsäure (22), die Vorrichtung umfassend:

- eine Heizeinrichtung mit einem Heizelement (10), wobei das Heizelement (10) mit einer Probenflüssigkeit (500) und/oder mit einer Reaktionslösung (400) in Kontakt bringbar ist und mit zumindest einer Funktionsnukleinsäure (14) konjugiert ist, wobei die zumindest eine Funktionsnukleinsäure (14) dazu ausgelegt ist, mit der Target-Nukleinsäure (22) zu hybridisieren und die Target-Nukleinsäure (22) an das Heizelement zu binden; und
- eine Bewegungseinrichtung (100, 200, 300), welche dazu ausgelegt ist, eine Relativbewegung zwischen dem zumindest einen Heizelement (10) und der Reaktionslösung (400) und/oder der Probenflüssigkeit (500) zu erzeugen, wenn das Heizelement (10) in Kontakt mit der Reaktionslösung (400) bzw. der Probenflüssigkeit (500) ist;

wobei die Vorrichtung (24) dazu eingerichtet ist, das Heizelement (10) von der Probenflüssigkeit (500) zu separieren und dadurch die Target-Nukleinsäure (22) aus der Probenflüssigkeit (500) zu extrahieren.

15. Vorrichtung (24) gemäß Anspruch 14, wobei das Heizelement (10) als elektrisches und insbesondere als resistives Heizelement ausgebildet ist und bevorzugt als Heizdraht (12) ausgebildet ist, und wobei die Heizeinrichtung vorzugsweise eine Steuereinheit aufweist, mittels der das eine Heizelement (10) oder die mehreren Heizelemente (10) beheizbar sind.

16. Vorrichtung (24) gemäß einem der Ansprüche 14 bis 15, wobei die Bewegungseinrichtung (100, 200, 300) dazu ausgelegt ist, das zumindest eine Heizelement (10) relativ zur Probenflüssigkeit (500) und/oder zur Reaktionslösung (400) zu bewegen und wobei die Bewegungseinrichtung (100, 200, 300) vorzugsweise zumindest einen mechanischen Aktor zur Bewegung des zumindest einen Heizelements (10) und/oder eine Steuereinheit zur Erzeugung von elektrischen Strompulsen und/oder Spannungspulsen zur Bestromung des zumindest einen Heizelements (10) aufweist.

17. Vorrichtung (24) gemäß einem der Ansprüche 14 bis 16, wobei die Bewegungseinrichtung (100, 200, 300) dazu ausgelegt ist, die Probenflüssigkeit (500) und/oder die Reaktionslösung (400) zumindest teilweise zu bewegen und wobei die Bewegungseinrichtung (100, 200, 300) vorzugweise zumindest eine Pumpe und/oder einen Schallwellengenerator und/oder einen Ultraschallwellengenerator und/oder einen extern treibbaren Aktor und/oder einen Treiber für einen externen treibbaren Aktor aufweist, um die Probenflüssigkeit (500) und/oder die Reaktionslösung (400) zumindest teilweise zu bewegen.

18. Vorrichtung (24) gemäß einem der Ansprüche 14 bis 17, wobei die Bewegungseinrichtung (100, 200, 300) dazu ausgelegt ist, ein Reaktionsgefäß (28), in welchem die Probenflüssigkeit (500) und/oder die Reaktionslösung (400) anordenbar sind, zumindest teilweise zu bewegen und wobei die Bewegungseinrichtung (100, 200, 300) vorzugweise zumindest einen Aktor aufweist, um das Reaktionsgefäß (28) zu schütteln und/oder zu zentrifugieren und/oder zu Drehen und/oder zu Schwenken und/oder zu Vortexen und/oder zu Kippen.

## Claims

1. Method for extracting a target nucleic acid (22) from a sample fluid (500), comprising the steps of:

- providing a heating device having a heating element (10) in contact with the sample liquid (500), wherein the heating element (10) is conjugated with at least one functional nucleic acid (14) and wherein the at least one functional nucleic acid (14) is designed to hybridize with the target nucleic acid (22) and to bind the target nucleic acid (22) to the heating element (10);
- generating a relative movement between the heating element (10) and the sample liquid (500);
- extracting the target nucleic acid (22) from the sample liquid (500) by separating the heating element (10) from the sample liquid (500).

2. Method for reproducing a target nucleic acid (22), comprising the steps of:

- extracting the target nucleic acid (22) from a sample liquid (500) by providing a heating device having a heating element (10) in contact with the sample liquid (500), wherein the heating element (10) is conjugated with at least one functional nucleic acid (14) and wherein the at least one functional nucleic acid (14) is designed to hybridize with the target nucleic acid (22) and to bind the target nucleic acid (22) to the heating element (10);
- reproducing the target nucleic acid (22) extracted from the sample liquid (500) by means of a polymerase chain reaction in a reaction solution (400) using the heating device;

wherein extracting the target nucleic acid (22) further comprises generating a relative movement between the heating element (10) and the sample liquid (500) and separating the heating element (10) from the sample liquid (500).

3. Method according to claim 2, wherein reproducing the target nucleic acid (22) further comprises generating a relative movement between the heating element (10) and the reaction solution (400).

4. Method according to any of the preceding claims, wherein the relative movement between the heating element (10) and the sample liquid (500) is generated when the heating element (10) is in contact with the sample liquid (500), and/or wherein the relative movement between the heating element (10) and the reaction solution (400) is generated at least partially during a denaturation step of the polymerase chain reaction.

5. Method according to any of the preceding claims, wherein at least during the generation of the relative movement between the heating element (10) and the sample liquid (500) and/or during the generation of the relative movement between the heating element (10) and the reaction solution (400), a temperature suitable for the hybridization of the target nucleic acid (22) with the functional nucleic acid (14) is at least temporarily provided at least at the location of the functional nucleic acid (14), and wherein the temperature is at least 20°C, preferably at least 30°C, more preferably at least 40°C, much more preferably at least 50°C and most preferably at least 60°C, and/or the temperature is no more than 90°C, preferably no more than 80°C, most preferably no more than 70°C, and wherein heat for providing the temperature is optionally at least partially provided by means of the heating element (10).

6. Method according to any of the preceding claims, wherein the functional nucleic acid (14) is designed as an extraction nucleic acid and/or has a capture nucleotide sequence which is at least partially complementary to the nucleotide sequence of the target nucleic acid (22) and is suitable for binding to the target nucleic acid (22).

7. Method according to any of the preceding claims, wherein the functional nucleic acid (14) has a nucleotide sequence which can be used as a primer sequence for the reproduction of the target nucleic acid (22).

8. Method according to any of the preceding claims, wherein the relative movement between the heating element (10) and the sample liquid (500) and/or the reaction solution (400) is at least partially carried out by moving the heating element (10).

9. Method according to claim 8, wherein the movement of the heating element (10) comprises oscillating and/or vibrating the heating element (10), and wherein the oscillating and/or vibrating of the heating element (10) is optionally caused by means of a magnetic field, wherein the magnetic field is preferably generated by an electric current flow in the heating element (10) and/or by an external magnetic field.

10. Method according to claim 9, wherein the oscillation and/or vibration of the heating element (10) is caused at least partially by a force applied by a mechanical actuator and/or by sound waves and/or ultrasonic waves.

11. Method according to any of the preceding claims, wherein the one or more heating elements (10) are designed as electrical and in particular as resistive heating elements (10) and are preferably designed as heating wire (12) or heating wires (12), and wherein the heating device optionally has a plurality of heating elements (10).

12. Method according to any of the preceding claims, wherein the heating element (10) is conjugated with a plurality of functional nucleic acids (14), wherein the functional nucleic acids (14) of the heating element (10) are of the same type or at least partially of different types.

13. Method according to claim 11, wherein each of the heating elements (10) is conjugated with a plurality of functional nucleic acids (14) of the same type, and the different heating elements (10) are preferably conjugated with functional nucleic acids (14) that are at least partially different from the functional nucleic acids (14) of the other heating elements (10).

14. Apparatus (24) for extracting and/or reproducing a target nucleic acid (22), the apparatus comprising:

- a heating device having a heating element (10), wherein the heating element (10) can be brought into contact with a sample liquid (500) and/or with a reaction solution (400) and is conjugated with at least one functional nucleic acid (14), wherein the at least one functional nucleic acid (14) is designed to hybridize with the target nucleic acid (22) and to bind the target nucleic acid (22) to the heating element; and
- a movement device (100, 200, 300) which is designed to generate a relative movement between the at least one heating element (10) and the reaction solution (400) and/or the sample liquid (500) when the heating element (10) is in contact with the reaction solution (400) or the sample liquid (500);

wherein the apparatus (24) is designed to separate the heating element (10) from the sample liquid (500) and, as a result, extract the target nucleic acid (22) from the sample liquid (500).

15. Apparatus (24) according to claim 14, wherein the heating element (10) is designed as an electrical, and in particular as a resistive, heating element and is preferably designed as a heating wire (12), and wherein the heating device preferably has a control unit, by means of which the one heating element (10) or the plurality of heating elements (10) can be heated.

16. Apparatus (24) according to either claim 14 or claim 15, wherein the movement device (100, 200, 300) is designed to move the at least one heating element (10) relative to the sample liquid (500) and/or to the reaction solution (400), and wherein the movement device (100, 200, 300) preferably has at least one mechanical actuator for moving the at least one heating element (10) and/or a control unit for generating electrical current pulses and/or voltage pulses for energizing the at least one heating element (10).

17. Apparatus (24) according to any of claims 14 to 16, wherein the movement device (100, 200, 300) is designed to at least partially move the sample liquid (500) and/or the reaction solution (400), and wherein the movement device (100, 200, 300) preferably has at least one pump and/or a sound wave generator and/or an ultrasonic wave generator and/or an externally drivable actuator and/or a driver for an externally drivable actuator, in order to at least partially move the sample liquid (500) and/or the reaction solution (400).

18. Apparatus (24) according to any of claims 14 to 17, wherein the movement device (100, 200, 300) is designed to at least partially move a reaction vessel (28) in which the sample liquid (500) and/or the reaction solution (400) can be arranged, and wherein the movement device (100, 200, 300) preferably has at least one actuator for shaking and/or centrifuging and/or rotating and/or pivoting and/or vortexing and/or tilting the reaction vessel (28).

## Revendications

1. Procédé destiné à l'extraction d'un acide nucléique cible (22) à partir d'un liquide échantillon (500), comprenant les étapes consistant à :

   - fournir un appareil de chauffage comportant un élément de chauffage (10) en contact avec le liquide échantillon (500), dans lequel l'élément de chauffage (10) est conjugué à au moins un acide nucléique fonctionnel (14) et dans lequel l'au moins un acide nucléique fonctionnel (14) est configuré pour s'hybrider avec l'acide nucléique cible (22) et pour lier l'acide nucléique cible (22) à l'élément de chauffage (10) ;
   - générer un mouvement relatif entre l'élément de chauffage (10) et le liquide échantillon (500) ;
   - extraire l'acide nucléique cible (22) du liquide échantillon (500) par une séparation de l'élément de chauffage (10) du liquide échantillon (500).

2. Procédé destiné à l'amplification d'un acide nucléique cible (22), comprenant les étapes consistant à :

   - extraire l'acide nucléique cible (22) d'un liquide échantillon (500) par la fourniture d'un appareil de chauffage comportant un élément de chauffage (10) en contact avec le liquide échantillon (500), dans lequel l'élément de chauffage (10) est conjugué à au moins un acide nucléique fonctionnel (14) et dans lequel l'au moins un acide nucléique fonctionnel (14) est configuré pour s'hybrider avec l'acide nucléique cible (22) et pour lier l'acide nucléique cible (22) à l'élément de chauffage (10) ;
   - amplifier l'acide nucléique cible (22) extrait du liquide échantillon (500) à l'aide d'une réaction en chaîne par polymérase dans une solution réactionnelle (400) à l'aide de l'appareil de chauffage ;

   dans lequel l'extraction de l'acide nucléique cible (22) comprend en outre une génération d'un mouvement relatif entre l'élément de chauffage (10) et le liquide échantillon (500) et une séparation de l'élément de chauffage (10) du liquide échantillon (500).

3. Procédé selon la revendication 2, dans lequel l'amplification de l'acide nucléique cible (22) comprend en outre une génération d'un mouvement relatif entre l'élément de chauffage (10) et la solution réactionnelle (400).

**4.** Procédé selon l'une des revendications précédentes, dans lequel la génération du mouvement relatif entre l'élément de chauffage (10) et le liquide échantillon (500) a lieu lorsque l'élément de chauffage (10) est en contact avec le liquide échantillon (500), et/ou dans lequel la génération du mouvement relatif entre l'élément de chauffage (10) et la solution réactionnelle (400) a lieu au moins partiellement pendant une étape de dénaturation de la réaction en chaîne par polymérase.

**5.** Procédé selon l'une des revendications précédentes, dans lequel, au moins pendant la génération du mouvement relatif entre l'élément de chauffage (10) et le liquide échantillon (500) et/ou pendant la génération du mouvement relatif entre l'élément de chauffage (10) et la solution réactionnelle (400), une température appropriée pour l'hybridation de l'acide nucléique cible (22) avec l'acide nucléique fonctionnel (14) est fournie au moins temporairement au moins à l'endroit de l'acide nucléique fonctionnel (14), et dans lequel la température est d'au moins 20 °C, de préférence d'au moins 30 °C, plus préférablement d'au moins 40 °C, encore plus préférablement d'au moins 50 °C et le plus préférablement d'au moins 60 °C, et/ou la température n'est pas supérieure à 90 °C, de préférence n'est pas supérieure à 80 °C, le plus préférablement n'est pas supérieure à 70 °C, et dans lequel une fourniture de chaleur pour la fourniture de la température est facultativement effectuée au moins partiellement à l'aide de l'élément de chauffage (10).

**6.** Procédé selon l'une des revendications précédentes, dans lequel l'acide nucléique fonctionnel (14) est réalisé sous forme d'un acide nucléique d'extraction et/ou présente une séquence nucléotidique de capture au moins partiellement complémentaire de la séquence nucléotidique de l'acide nucléique cible (22) et apte à se lier à l'acide nucléique cible (22).

**7.** Procédé selon l'une des revendications précédentes, dans lequel l'acide nucléique fonctionnel (14) présente une séquence nucléotidique utilisable comme séquence d'amorce pour l'amplification de l'acide nucléique cible (22).

**8.** Procédé selon l'une des revendications précédentes, dans lequel le mouvement relatif entre l'élément de chauffage (10) et le liquide échantillon (500) et/ou la solution réactionnelle (400) est effectué au moins en partie par un mouvement de l'élément de chauffage (10).

**9.** Procédé selon la revendication 8, dans lequel le mouvement de l'élément de chauffage (10) comprend une oscillation et/ou une vibration de l'élément de chauffage (10), et dans lequel l'oscillation et/ou la vibration de l'élément de chauffage (10) est éventuellement provoquée à l'aide d'un champ magnétique, dans lequel le champ magnétique est de préférence généré par un flux de courant électrique dans l'élément de chauffage (10) et/ou par un champ magnétique externe.

**10.** Procédé selon la revendication 9, dans lequel l'oscillation et/ou la vibration de l'élément de chauffage (10) est provoquée au moins en partie par l'application d'une force par un actionneur mécanique et/ou par des ondes sonores et/ou des ondes ultrasonores.

**11.** Procédé selon l'une des revendications précédentes, dans lequel l'un ou les plusieurs éléments de chauffage (10) sont réalisés sous forme d'éléments de chauffage (10) électriques et en particulier résistifs et sont de préférence réalisés sous forme de fil de chauffage (12) ou de fils de chauffage (12), et dans lequel l'appareil de chauffage présente éventuellement plusieurs éléments de chauffage (10).

**12.** Procédé selon l'une des revendications précédentes, dans lequel l'élément de chauffage (10) est conjugué à une pluralité d'acides nucléiques fonctionnels (14), dans lequel les acides nucléiques fonctionnels (14) de l'élément de chauffage (10) sont réalisés de même nature ou au moins partiellement de nature différente.

**13.** Procédé selon la revendication 11, dans lequel chacun des éléments de chauffage (10) est conjugué respectivement avec une pluralité d'acides nucléiques fonctionnels (14) de même type et les différents éléments de chauffage (10) sont conjugués de préférence avec des acides nucléiques fonctionnels (14) qui sont au moins partiellement différents des acides nucléiques fonctionnels (14) des autres éléments de chauffage (10).

**14.** Dispositif (24) destiné à l'extraction et/ou l'amplification d'un acide nucléique cible (22), le dispositif comprenant :

  - un appareil de chauffage comportant un élément de chauffage (10), dans lequel l'élément de chauffage (10) peut être mis en contact avec un liquide échantillon (500) et/ou avec une solution réactionnelle (400) et est conjugué à au moins un acide nucléique fonctionnel (14), dans lequel l'au moins un acide nucléique fonctionnel (14) est configuré pour s'hybrider avec l'acide nucléique cible (22) et pour lier l'acide nucléique cible (22) à l'élément de chauffage ; et
  - un dispositif de mouvement (100, 200, 300) qui est configuré pour générer un mouvement relatif entre l'au moins un élément de chauffage (10) et la solution réactionnelle (400) et/ou le liquide échantillon (500) lorsque l'élément de chauffage (10) est en contact avec la solution réactionnelle (400) ou le liquide échantillon (500), respectivement ;

dans lequel le dispositif (24) est conçu pour séparer l'élément de chauffage (10) du liquide échantillon (500) et pour extraire ainsi l'acide nucléique cible (22) du liquide échantillon (500).

15. Dispositif (24) selon la revendication 14, dans lequel l'élément de chauffage (10) est réalisé sous la forme d'un élément de chauffage électrique et en particulier résistif et est réalisé de préférence sous la forme d'un fil de chauffage (12), et dans lequel l'appareil de chauffage présente de préférence une unité de commande à l'aide de laquelle l'un élément de chauffage (10) ou les plusieurs éléments de chauffage (10) peuvent être chauffés.

16. Dispositif (24) selon l'une des revendications 14 à 15, dans lequel le dispositif de mouvement (100, 200, 300) est configuré pour le mouvement l'au moins un élément de chauffage (10) par rapport au liquide échantillon (500) et/ou à la solution réactionnelle (400) et dans lequel le dispositif de mouvement (100, 200, 300) présente de préférence au moins un actionneur mécanique pour le mouvement de l'au moins un élément de chauffage (10) et/ou une unité de commande pour la génération d'impulsions de courant électrique et/ou d'impulsions de tension pour alimenter l'au moins un élément de chauffage (10).

17. Dispositif (24) selon l'une des revendications 14 à 16, dans lequel le dispositif de mouvement (100, 200, 300) est configuré pour le mouvement au moins partiel du liquide échantillon (500) et/ou de la solution réactionnelle (400) et dans lequel le dispositif de mouvement (100, 200, 300) présente de préférence au moins une pompe et/ou un générateur d'ondes sonores et/ou un générateur d'ondes ultrasonores et/ou un actionneur pouvant être entraîné de manière externe et/ou un pilote pour un actionneur pouvant être entraîné de manière externe pour le mouvement au moins partiel du liquide échantillon (500) et/ou de la solution réactionnelle (400).

18. Dispositif (24) selon l'une des revendications 14 à 17, dans lequel le dispositif de mouvement (100, 200, 300) est configuré pour le mouvement au moins partiel d'un récipient de réaction (28) dans lequel le liquide échantillon (500) et/ou la solution réactionnelle (400) peuvent être disposés, et dans lequel le dispositif de mouvement (100, 200, 300) présente de préférence au moins un actionneur pour agiter et/ou centrifuger et/ou tourner et/ou faire pivoter et/ou vortexer et/ou basculer le récipient de réaction (28).

Fig. 1A

Fig. 1B

**Fig. 2A**

**Fig. 2B**

Fig. 2C

Fig. 2D

Fig. 3A

Fig. 3B

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016120124 A1
- WO 2018073435 A1
- WO 9525592 A
- DE 102013215168 A1
- DE 102016120124

**In der Beschreibung aufgeführte Nicht-Patentliteratur**

- **AUFSATZ VON D. RENNEBERG** ; **C.J. LEUMANN**. Watson-Crick base-pairing properties of Tricyclo-DNA. *J. Am. Chem. Soc.*, 2002, vol. 124, 5993-6002